# FASCICULE DE BREVET EUROPEEN

(11) **EP 0 502 786 B1**
(45) Date de publication et mention de la délivrance du brevet: **24.04.1996**
(21) Numéro de dépôt: 92400565.5
(22) Date de dépôt: 04.03.1992
(51) Int. Cl.: C07D 403/06, C07D 403/14, C07D 471/04, C07D 487/10

(54) **Procédé de préparation de dérivés d'aryl (ou hétéroaryl)-pipérazinyl-butyl-azoles**
Verfahren zur Herstellung von Aryl(oder Heteroaryl)-piperazin-1-butyl-azol-derivaten
Process for the preparation of aryl(or heteroaryl)-piperazin-1-butyl-azole derivatives

(30) Priorité: 07.03.1991 FR 9102735
(43) Date de publication de la demande: 09.09.1992
(73) Titulaire: LABORATORIOS DEL DR. ESTEVE, S.A., E-08026 Barcelona (ES)
(72) Inventeur: Merce-Vidal, Ramon, E-08014 Barcelone (ES); Frigola-Constansa, Jordi, E-08013 Barcelone (ES); Pares-Corominas, Juan, E-08025 Barcelone (ES)
(74) Mandataire: Ahner, Francis

(56) Documents cités:
- CHEMICAL ABSTRACTS, vol. 115, no. 17, 28 Octobre 1991, Columbus, Ohio, US; abstract no. 183160N, W. MALINKA: 'synthesis and properties of 2H-2-(4 substituted-1-piperazinylalkyl)-4,6-dimethyl-3 oxo-2,3-dihydroisothiazolo(5,4- b)pyridines'
- CHEMICAL ABSTRACTS, vol. 70, no. 13, 31 Mars 1969, Columbus, Ohio, US; abstract no. 57766D, E.L.STOGRYN: 'Antimalarials related to 2-bromo-4 ,5-dimethoxy-N,N- bis(diethylaminoethyl)aniline'

## Description

La présente invention concerne un procédé pour la préparation de dérivés d'aryl (ou hétéroaryl)-pipérazinyl-butyl-azoles, doté d'un excellent rendement (supérieur à 80%) et consuisant à des produits très purs. Ces dérivés répondent à la formule générale I 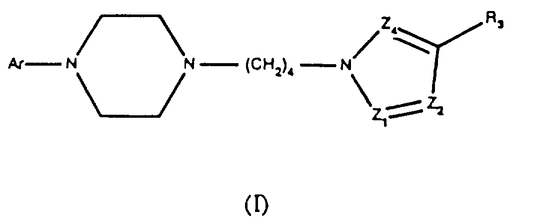 dans laquelle :
Ar représente un radical aromatique azote ou non, choisi parmi le radical phényle, la 2-pyrimidine, le 2-N-méthylimidazole et le 3-(1,2-benzothiazole)
Z₁ représente un atome d'azote ou un atome de carbone substitué ou non qu'on peut représenter par: C-R₁
Z₂ représente un atome d'azote ou un atome de carbone substitué ou non qu'on peut représenter par: C-R₂
Z₄ représente un atome d'azote ou un atome de carbone substitué ou non qu'on peut représenter par: C-R₄
et R₁, R₂, R₃ et R₄, identiques ou différents, pouvant également former une partie d'un autre cycle, aromatique ou non, représentent un atome d'hydrogène, un halogène, un radical alkyle inférieur, un radical nitro, un radical hydroxy, un radical alcoxy, un radical cyano, un radical carboxylique, un radical carboxamido, un radical carboxylate d'alkyle, un radical aryle, un radical sulfonique, un radical sulfonamido, un radical benzamido, un radical amino.

Les dérivés de 1-{4-[4-[aryl (ou hétérnaryl)]-1-pipérazinyl]-butyl}-1*H-*azole de formule générale I, sont des agents avec activité pharmacologique sur le système nerveux central, en particulier ils présentent des activités anxiolytique et tranquillisante, ainsi qu'antidépressive, dans l'inhibition du syndrome d'abstinence et dans les troubles associés à la cognition et sur le système cardiovasculaire en particulier avec activité antihypertensive, décrits dans nos propres travaux (Brevet français, FR 2642759, et demande de brevet français FR 91/00923).

Dans les techniques antérieures précédemment citées, on préparait les dérivés de 1-{4-[4-[aryl (ou hétéroaryl)]-1-pipérazinyl]-butyl}-1*H*-azole moyennant une des méthodes suivantes:

Par réaction d'un composé de formule générale II 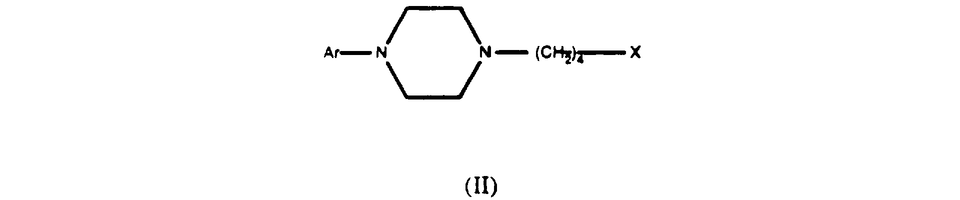 dans laquelle
Ar a les significations mentionnées précédemment et X représente un atome d'halogène, ou un groupe partant choisi parmi le tosyloxy ou le mésiloxy, avec un composé de formule générale III 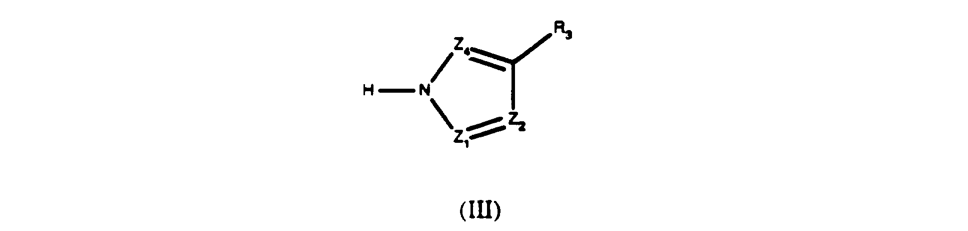 dans laquelle
Z₁, Z₂, Z₄ et R₃ ont les significations mentionnées précédemment, ou bien par réaction d'un composé de formule générale IV 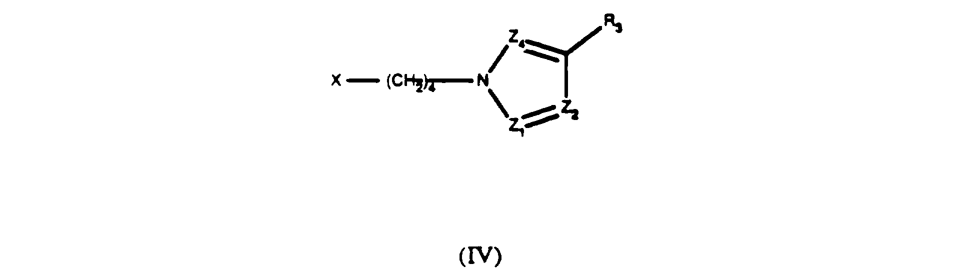 dans laquelle
Z₁, Z₂, Z₄, R₃ et X ont les significations mentionnées précédemment, avec un composé de formule générale V. 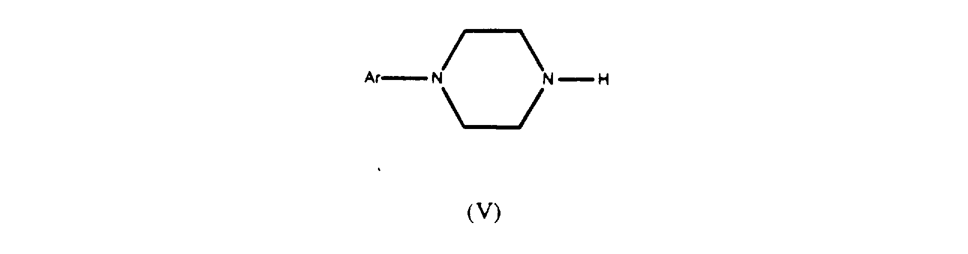 dans laquelle
Ar a les significations mentionnées précédemment, ou par réaction d'un composé de formule générale VI 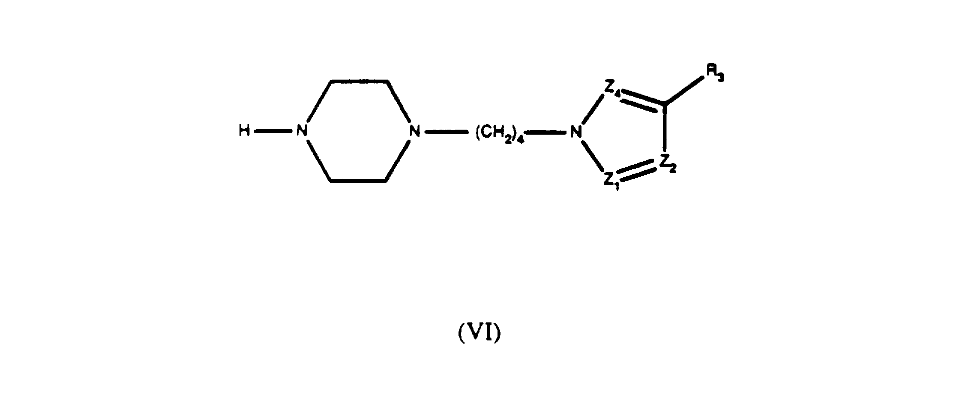 dans laquelle
Z₁, Z₂, Z₄ et R₃ ont les significations mentionnées précédemment, avec un composé de formule générale VII.

Aᵣ-X (VII)

dans laquelle
Ar et X ont les significations mentionnées précédemment, ou bien par réaction d'un composé de formule générale VIII 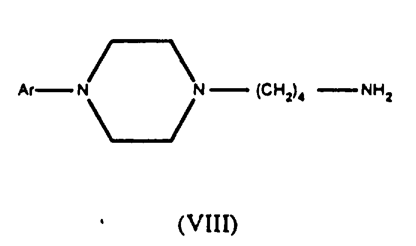 dans laquelle
Ar a les significations mentionnées précédemment, avec le 2,5-diméthoxytétrahydrofurane.

La présente invention concerne un nouveau procédé pour la préparation des dérivés de formule générale I précédemment définie qui permet l'amélioration des rendements d'obtention de ces produits ainsi que la mise en oeuvre industrielle. Conformément à l'invention, on prépare les dérivés de formule générale 1 en faisant réagir un composé de formule générale IX 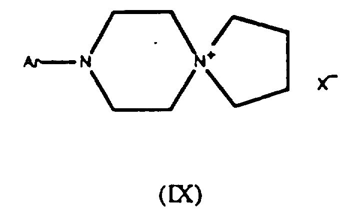 dans laquelle
Ar a les significations mentionnées précédemment et X représente un atome d'halogène, avec un composé de formule générale III 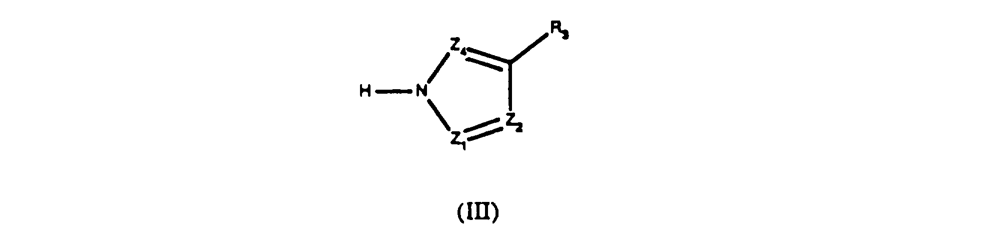 dans laquelle
Z₁, Z₂, Z₄ et R₃ ont les significations mentionnées précédemment.

Les températures les plus adéquates varient entre la température ambiante et la température de reflux du solvant, de préférence entre 80°C et 160°C, et le temps réactionnel est compris entre l'heure et 24 heures.

En opérant de la sorte, on obtient, conformément à la présente invention des dérivés de formule générale I ayant un degré de pureté très élevé. Ces dérivés sont en outre obtenus par un procédé de mise en oeuvre industrielle très simple et conduisant à un rendement très élevé.

Les produits de départ de formule générale IX sont preparés selon des méthodes décrites, par exemple: Yevich J.P. et al., J. Med. Chem., 1986, 29, 359.

Un grand nombre de composés ont été préparés par le procédé de l'invention. Leurs données physiço-chimiques ont été regroupées dans les tableaux I à III. La préparation de quelques dérivés de formule générale I a été décrite ci-après de façon détaillée.

### Exemple 11

### Préparation de 4,5-dichloro-2-méthyl-1-{4-[4-(2-pyrimidinyl)-1-pipérazinyl]-butyl}-1H-imidazole.

On chauffe à 130-135° pendant 14 heures un mélange de 450 g (1,5 moles) de bromure de 8-(2-pyrimidinyl)-8-aza-5-azoniaspiro[4,5]decane, 225 g (1,5 moles) de 4,5-dicloro-2-méthylimidazole et 300 g (2,25 moles) de carbonate de potassium, dans 2 l de diméthytformamide. On évapore sous vide, on ajoute du chloroforme, on lave à l'eau, on sèche sur sulfate de sodium, on évapore sous vide et on obtient 503 g (91%) de 4,5-dichloro-2-méthyl-1-{4[4-(2-pyrimidinyl)-1-pipérazinyl]-butyl}-1H-imidazole, sous une forme liquide.

Les données spectroscopiques pour l'identification de ce produit sont exposées dans le tableau I.

### Exemple 16

### Préparation de 1-{4-[4-(2-pyrimidinyl)-1-pipérazinyl]-butyl}-1H-benzimidazole.

On chauffe à 140-145°C pendant 14 heures un mélange de 450 g (1,50 moles) de bromure de 8-(2-pyrimidinyl)-8-aza-5-azoniaspiro[4,5]décane, 177 g (1.50 moles) de benzimidazole et 307 g (2,25 moles) de carbonate de potassium, dans 2 l de diméthylformamide. On évapore sous vide, on ajoute du chloroforme, on lave à l'eau. On sèche sur sulfate de sodium, on évapore sous vide et on obtient 457 g (91%) de 1-{4-[4-(2-pyrimidinyl)-1-pipérazinyl]-butyl}-1*H*-benzimidazole, avec un point de fusion de 85-88°C.

Les données spectroscopiques pour l'identification de ce produit sont exposées dans le tableau I.

### Exemple 27

### Préparation de 1-{4-[4-(2-pyrimidinyl)-1-pipérazinyl]-butyl}-1H-pirazole.

On chauffe à 140°C pendant 14 heures un mélange de 730 g (3,59 moles) de bromure de 8-(2-pyrimidinyl)-8-aza-5-azoniaspiro[4,5]decane, 275 g (4,05 moles) de pyrazole et 745 g (5,4 moles) de carbonate de potassium, dans 3 l de diméthylformamide. On évapore sous vide, on ajoute du chloroforme, on lave à l'eau, on sèche sur sulfate de sodium, on évapore sous vide et on obtient 650 g (94%) de 1-{4-[4-(2-pyrimidinyl)-1-pipérazinyl]-butyl}-1*H*-pyrazole, sous une forme liquide.

Les données spectroscopiques pour l'identification de ce produit sont exposées dans le tableau I.

### Exemple 34

### Préparation de 4-chloro-1-{4-[4-(2-pyrimidinyl)-1-pipérazinyl]-butyl}-1H-pirazole.

On chauffe à 120-125°C pendant 22 heures un mélange de 8,5 kg (28,41 moles) de bromure de 8-(2-pyrimidinyl)-8-aza-5-azoniaspiro[4,5]decane, 3,5 kg (34,14 moles) de 4-chloropyrazole et 5,5 kg (39,8 moles) de carbonate de potassium, dans 25,5 1 de diméthylformamide. On évapore sous vide, on ajoute du chloroforme, on lave à l'eau, on sèche sur sulfate de sodium, on évapore sous vide et on obtient 7,94 kg (87%) de 4-chloro-1-{4-[4-(2-pyrimidinyl)-1-pipérazinyl]-butyl}-1*H*-pyrazole, sous une forme liquide.

Les données spectroscopiques pour l'identification de ce produit sont exposées dans le tableau I.

### Exemple 77

### Préparation de 4,5 -dichloro-2-méthyl-1-{4-[4(2-méthoxyphényl)-1-pipérazinyl]-butyl}-1H-imidazole.

On chauffe à 130°C pendant 20 heures un mélange de 130 g (0,4 moles) de bromure de 8-(2-méthoxyphényl)-8-aza-5-azoniaspiro[4,5]decane, 66 g (0,44 moles) de 4,5-dichloro-2-méthylimidazole et 8.2 g (0,6 moles) de carbonate de potassium, dans 700 ml de diméthylformamide. On évapore sous vide, on ajoute du chloroforme, on lave à l'eau, on sèche sur sulfate de sodium, on évapore sous vide et on obtient 130 g (82%) de 4,5-dichloro-2-méthyl-1-{4-[4-(2-méthoxyphényl)-1-pipérazinyl]-butyl}-1*H*-imidazole avec un point de fusion de 82-83°C.

Les données spectroscopiques pour l'identification de ce produit sont exposées dans le tableau II.

### Exemple 88

### Préparation de 4-chloro-1-{4-[4-(3-(1,2-benzisothiazolyl))-1-pipérazinyl]-butyl}-1H-pirazole.

On chauffe à 140°C pendant 18 heures un mélange de 67 g (0,19 moles) de bromure de 8-(1,2-benzisothiazole-3-yl)-8-aza-5-azoniaspiro[4,5]décante, 20,5 g (0.2 moles) de 4-chloropyrazole et 41 g (0.3 moles) de carbonate de potassium, dans 300 ml de diméthylformamide. On évapore sous vide, on ajoute du chloroforme, on lave à l'eau, on sèche sur sulfate de sodium, on évapore sous vide et on obtient 60 g (80%) de 4-chloro-1-{4-[4-(3-(1,2-benzisothiazolyl))-1-pipérazinyl]-butyl}-1*H*-pirazole, sous une forme liquide.

Les données spectroscopiques pour l'identification de ce produit sont exposées dans le tableau III.

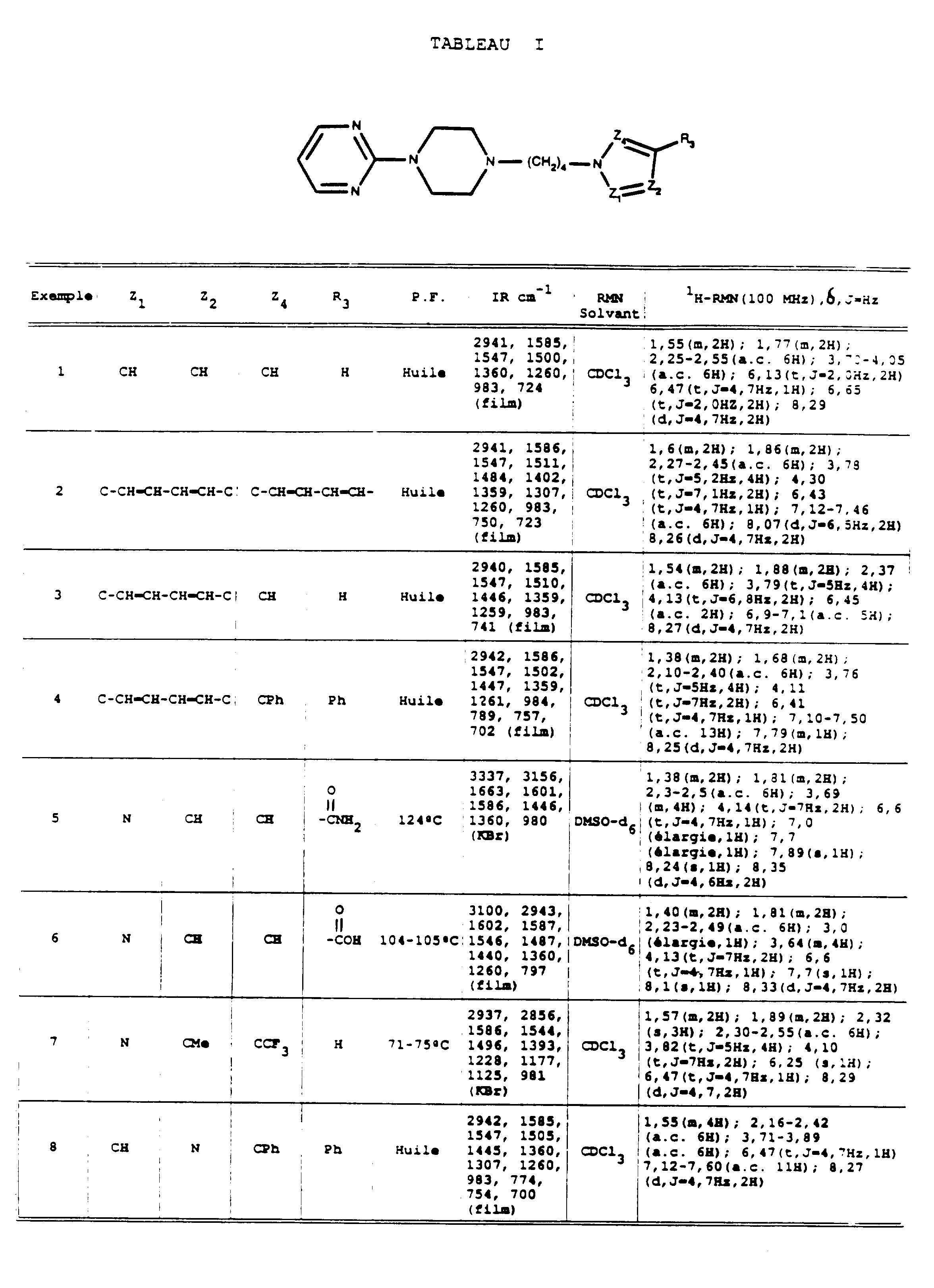

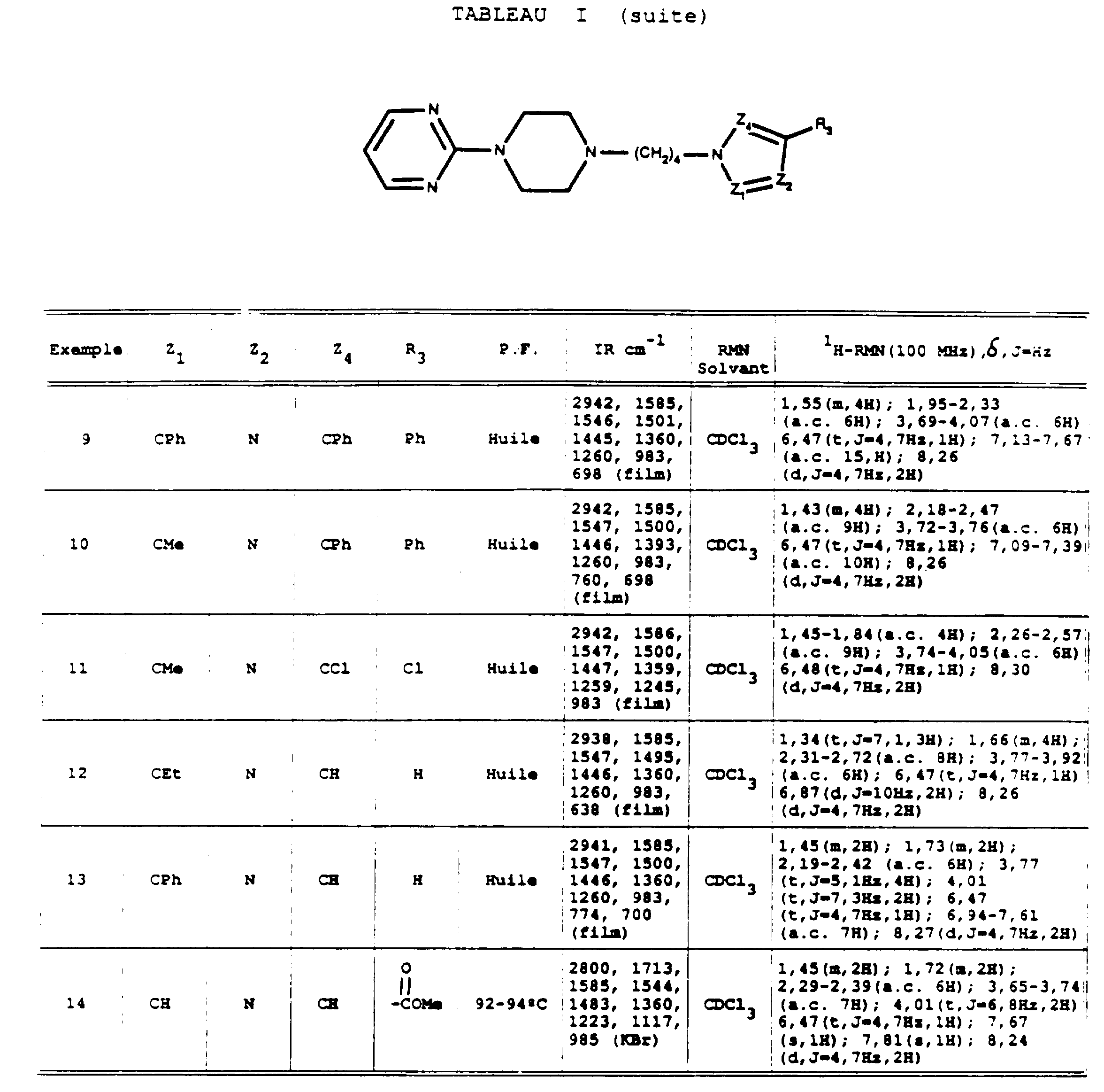

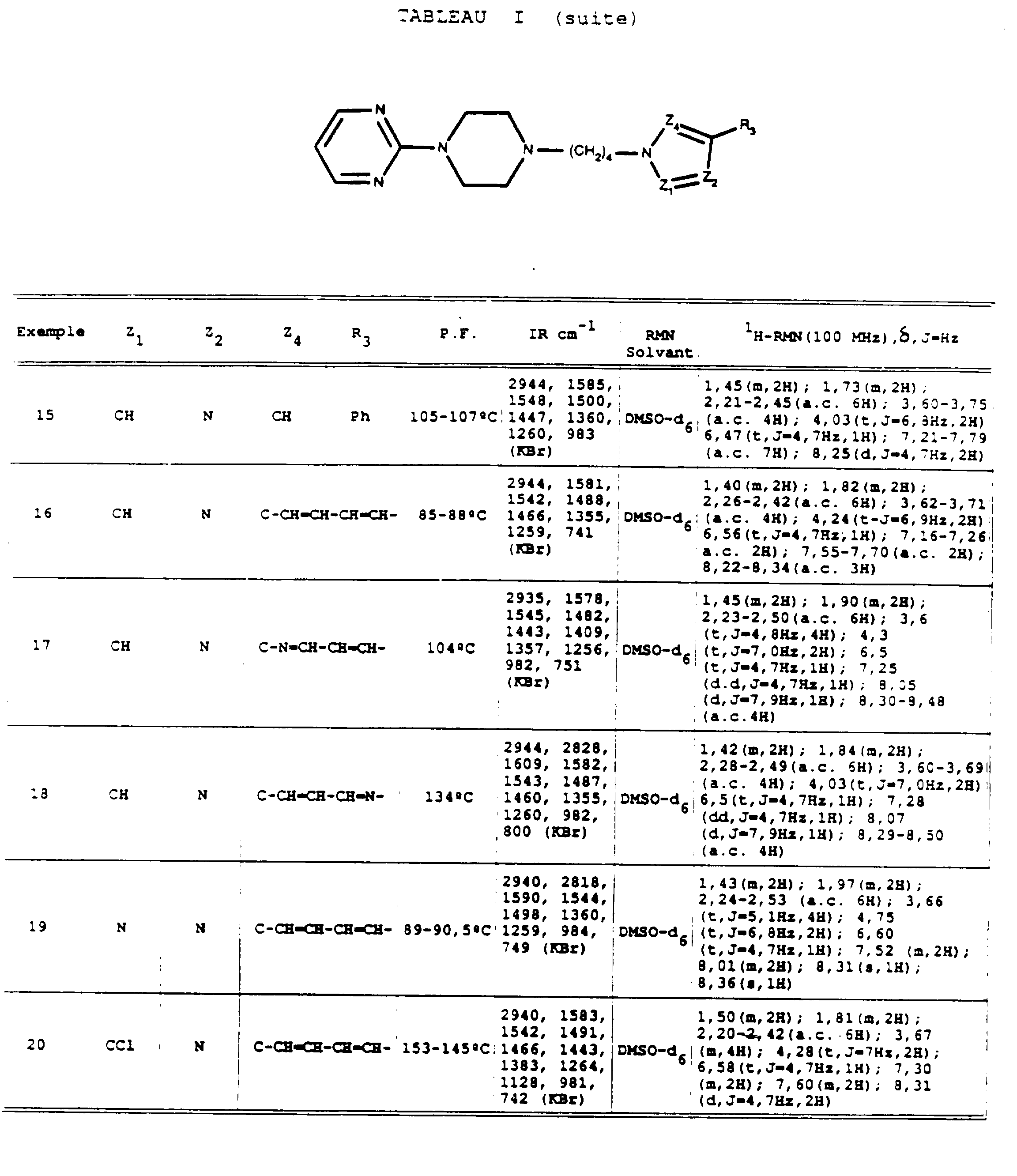

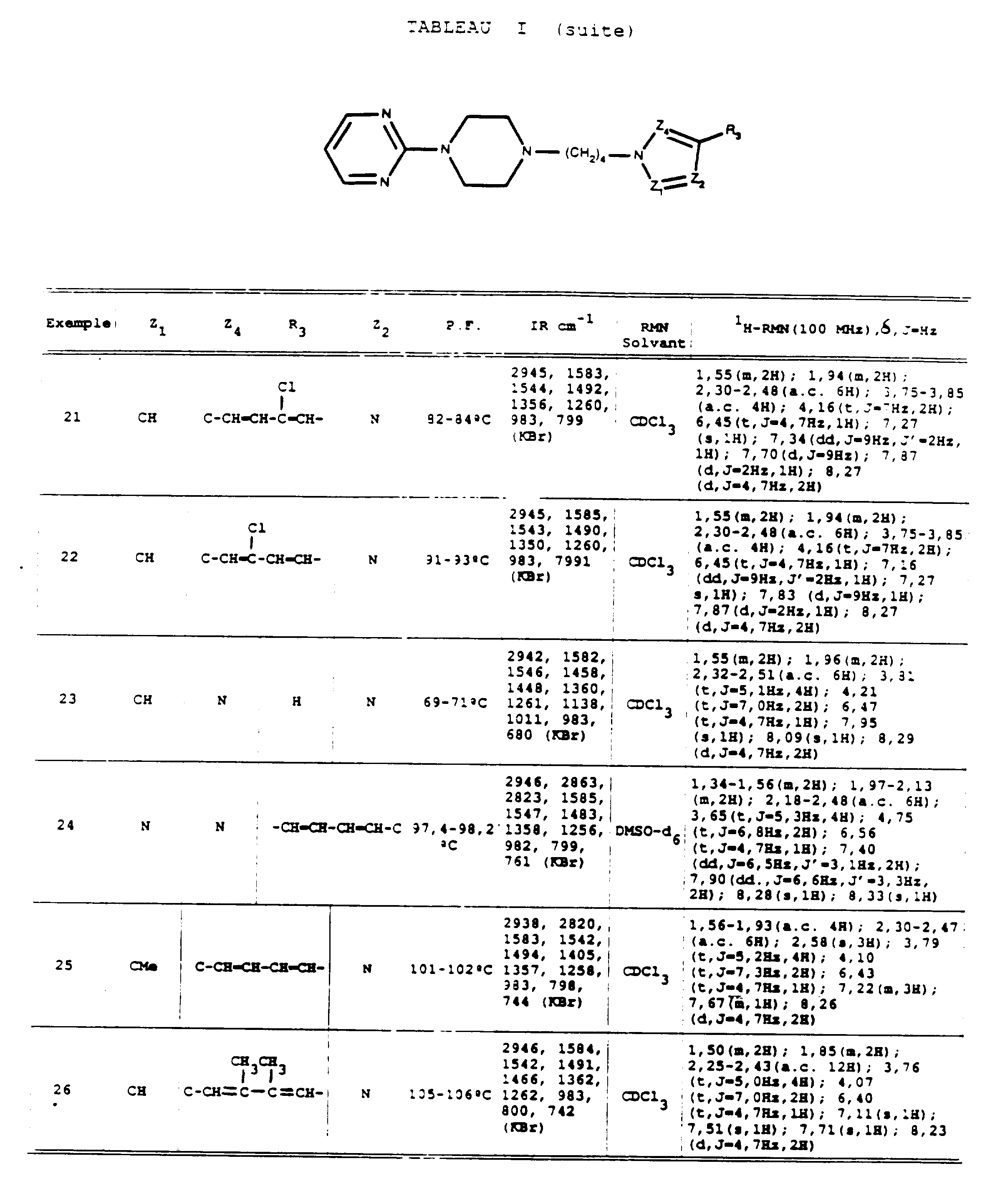

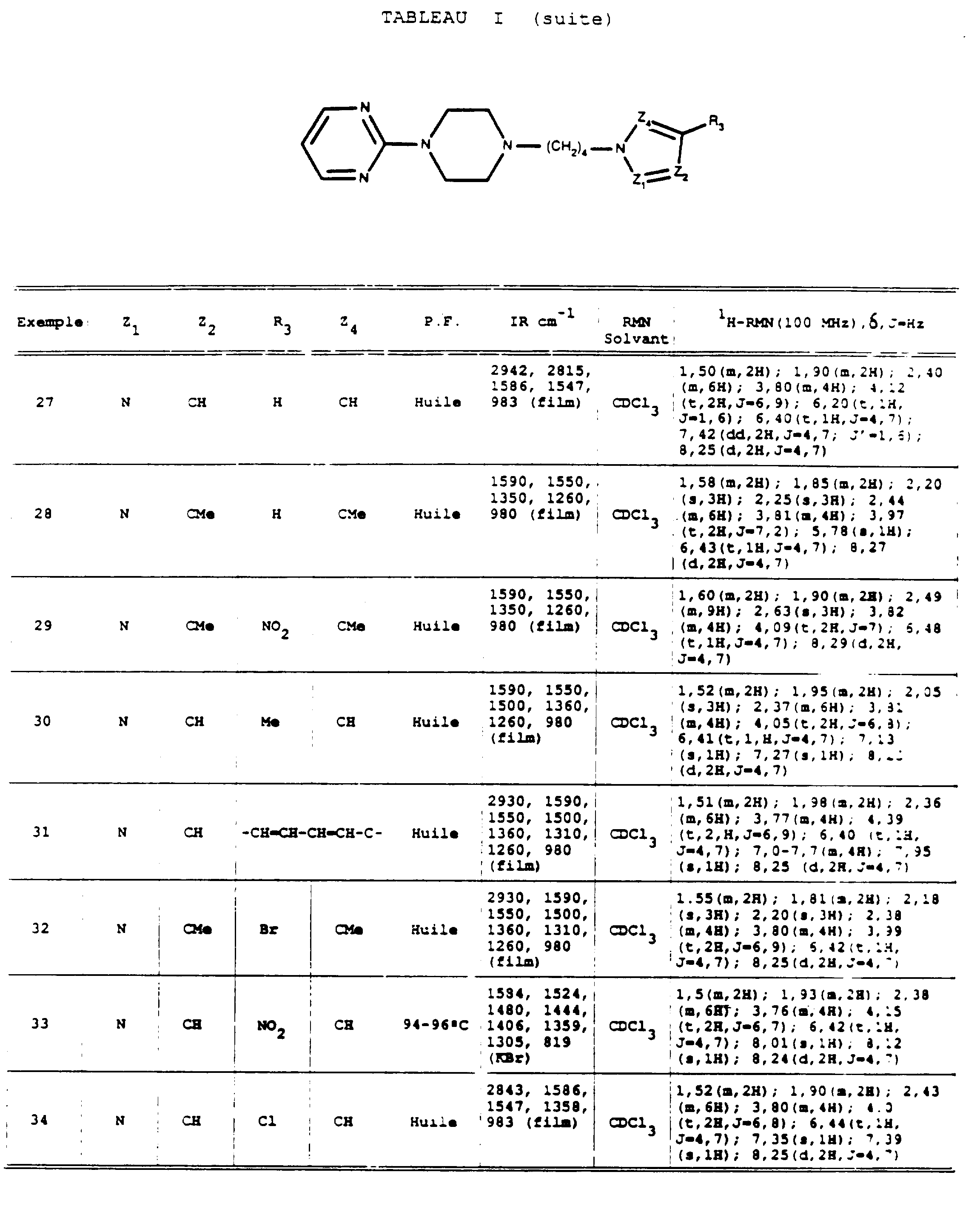

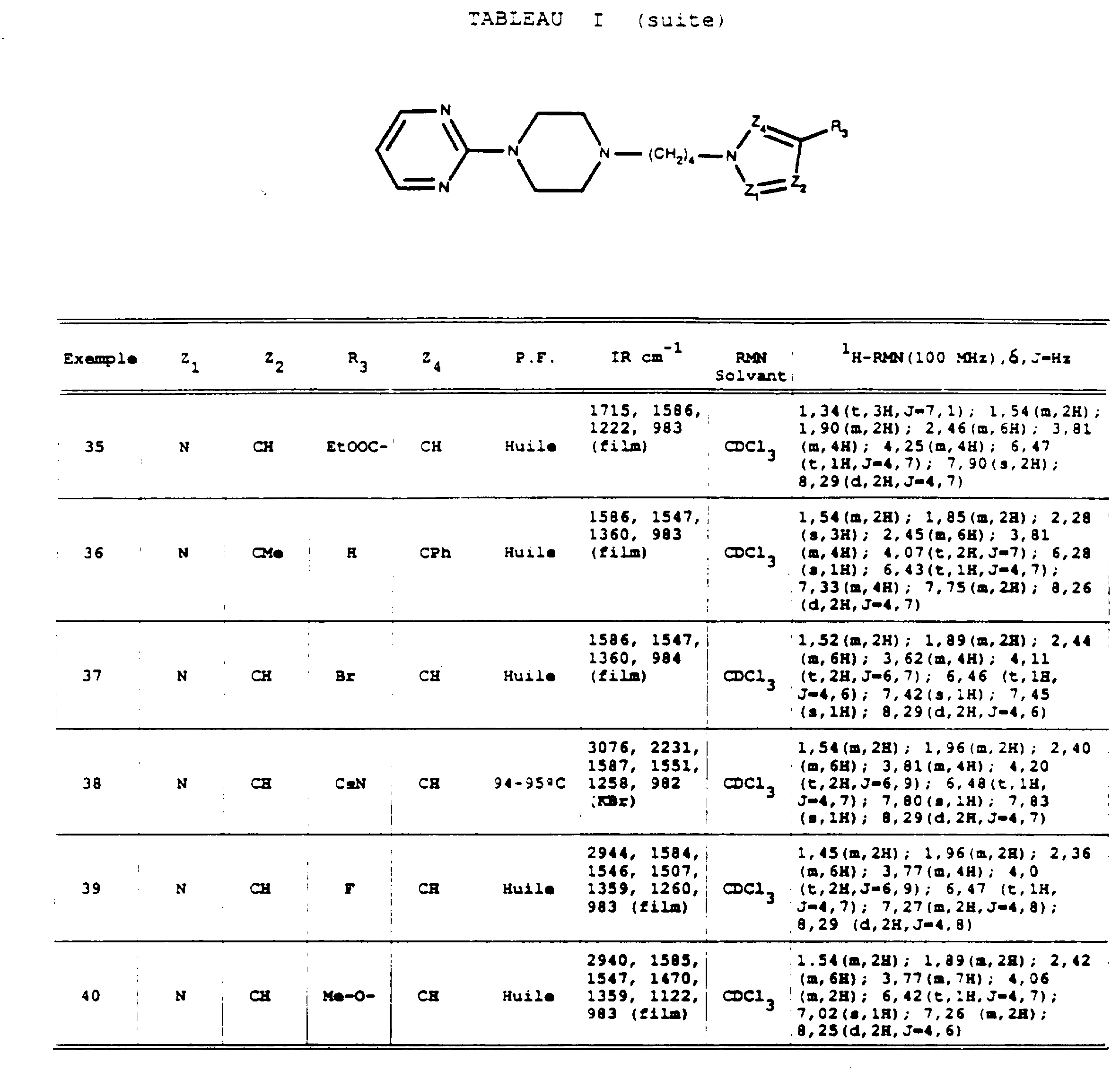

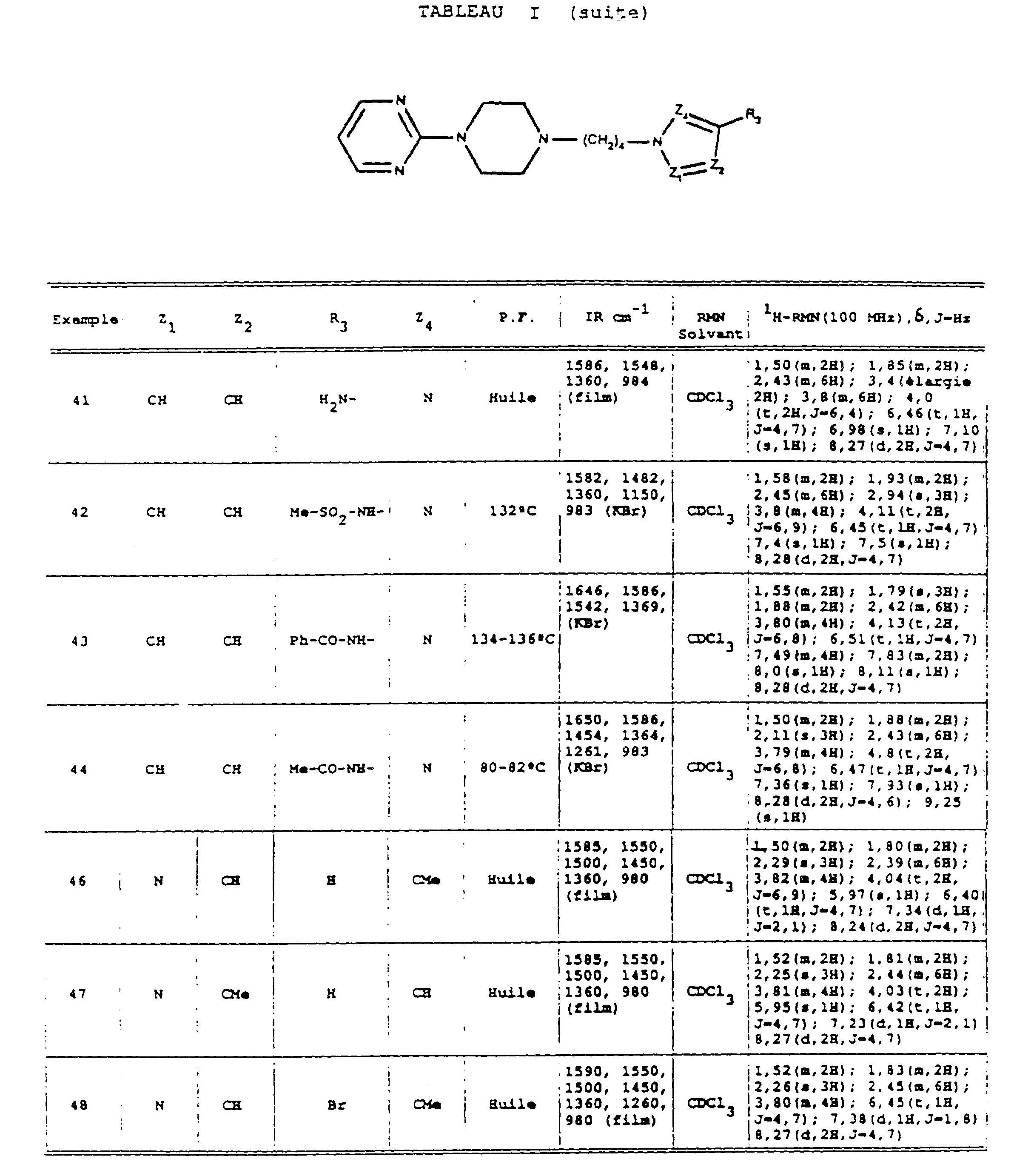

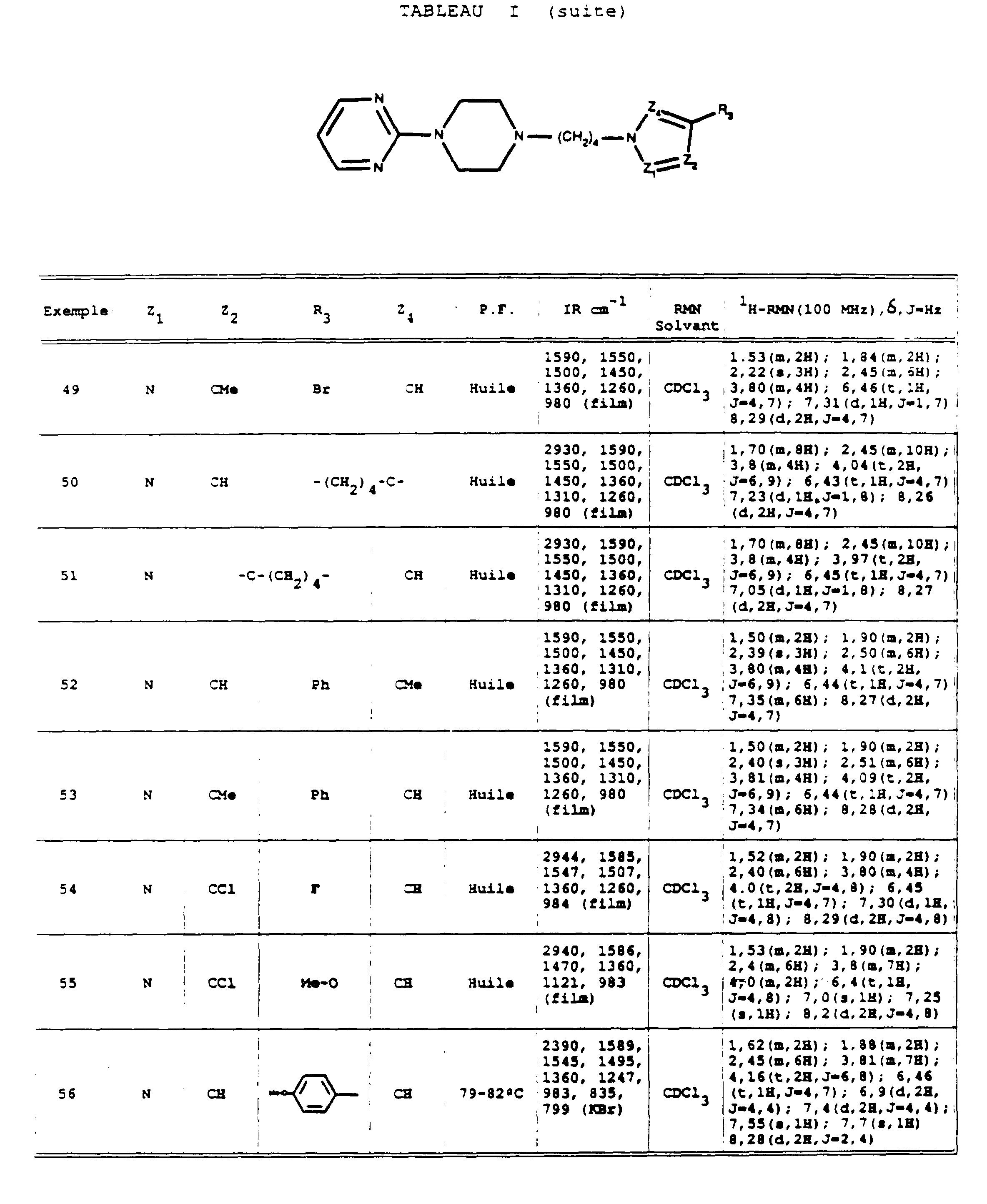

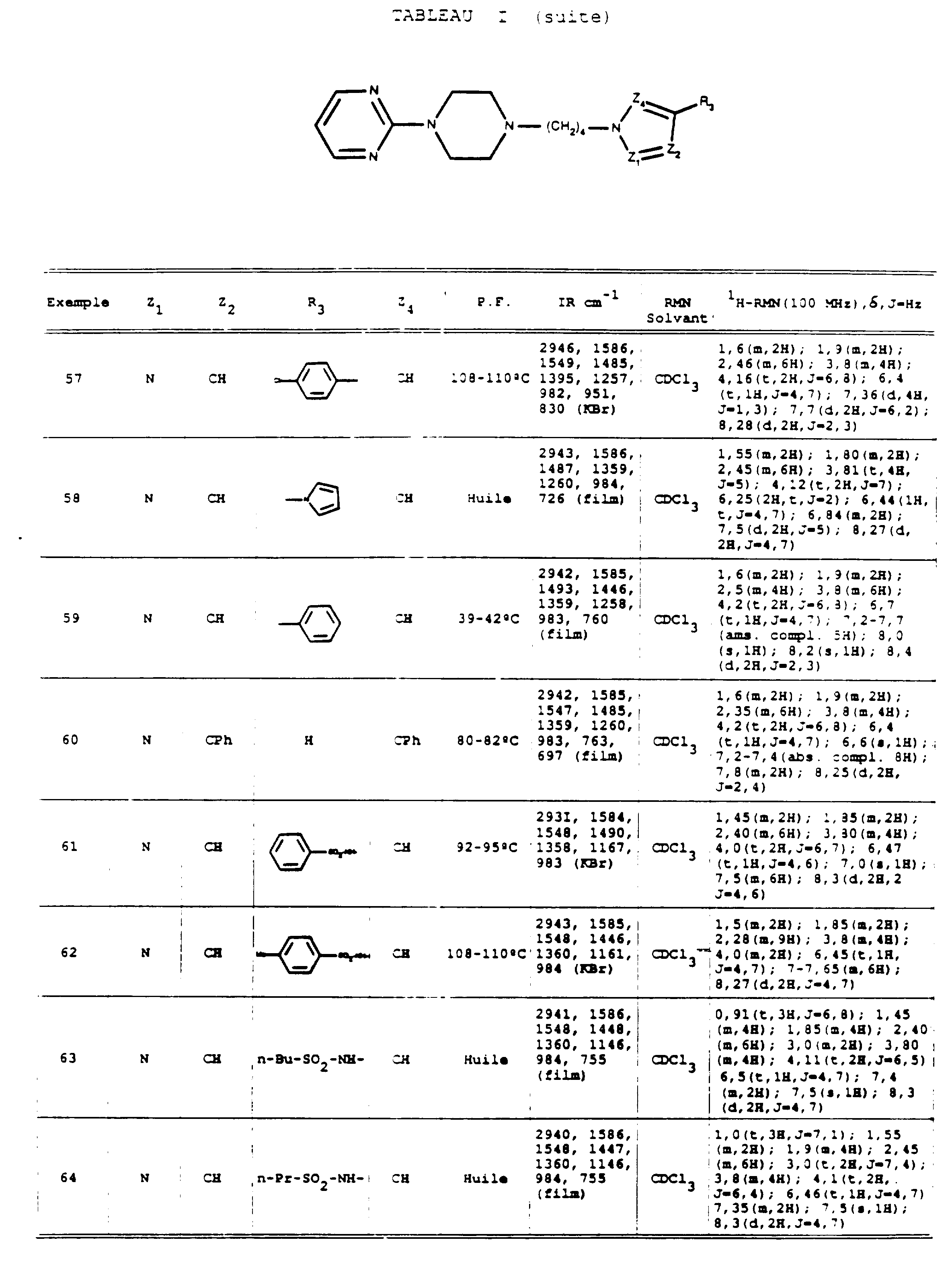

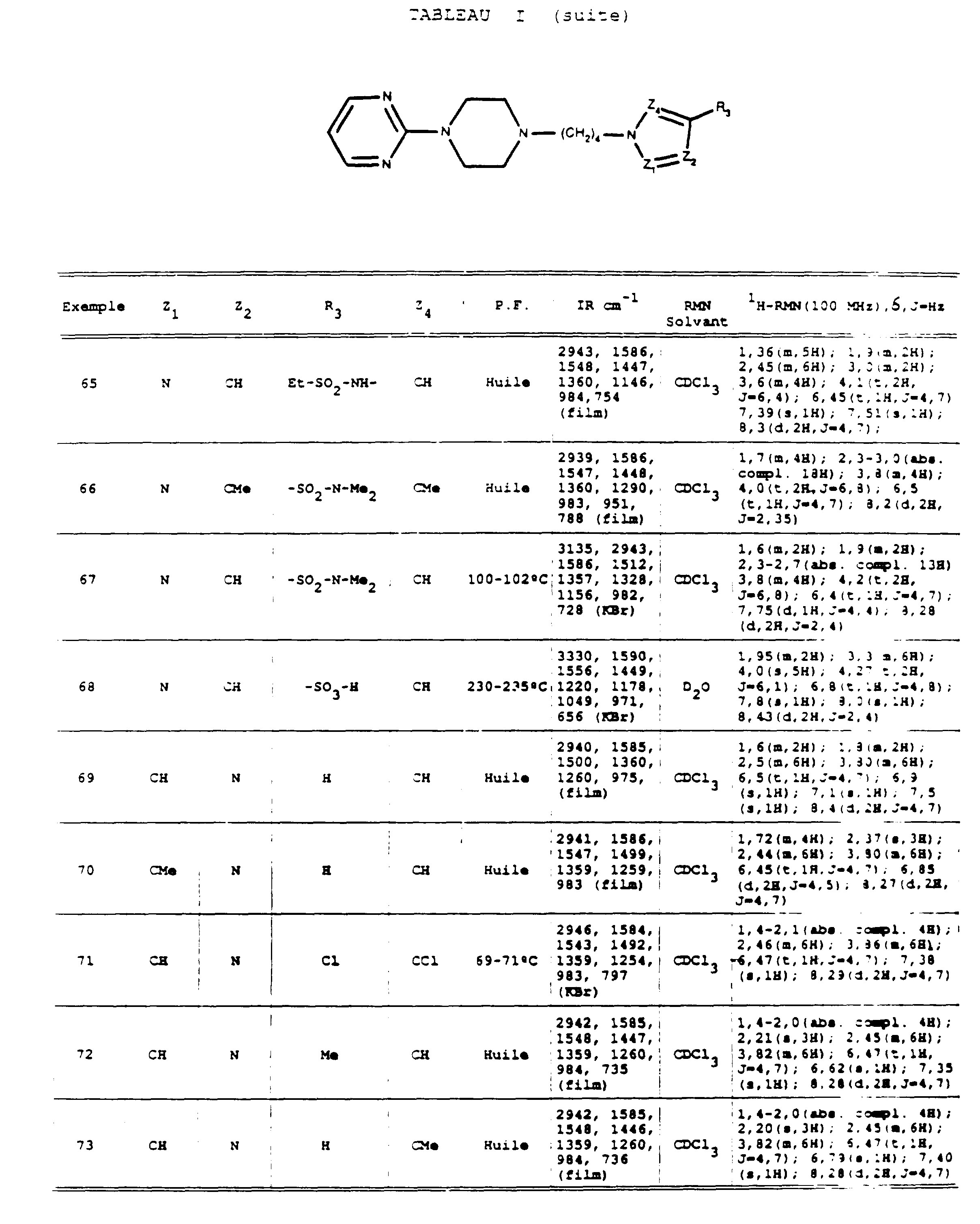

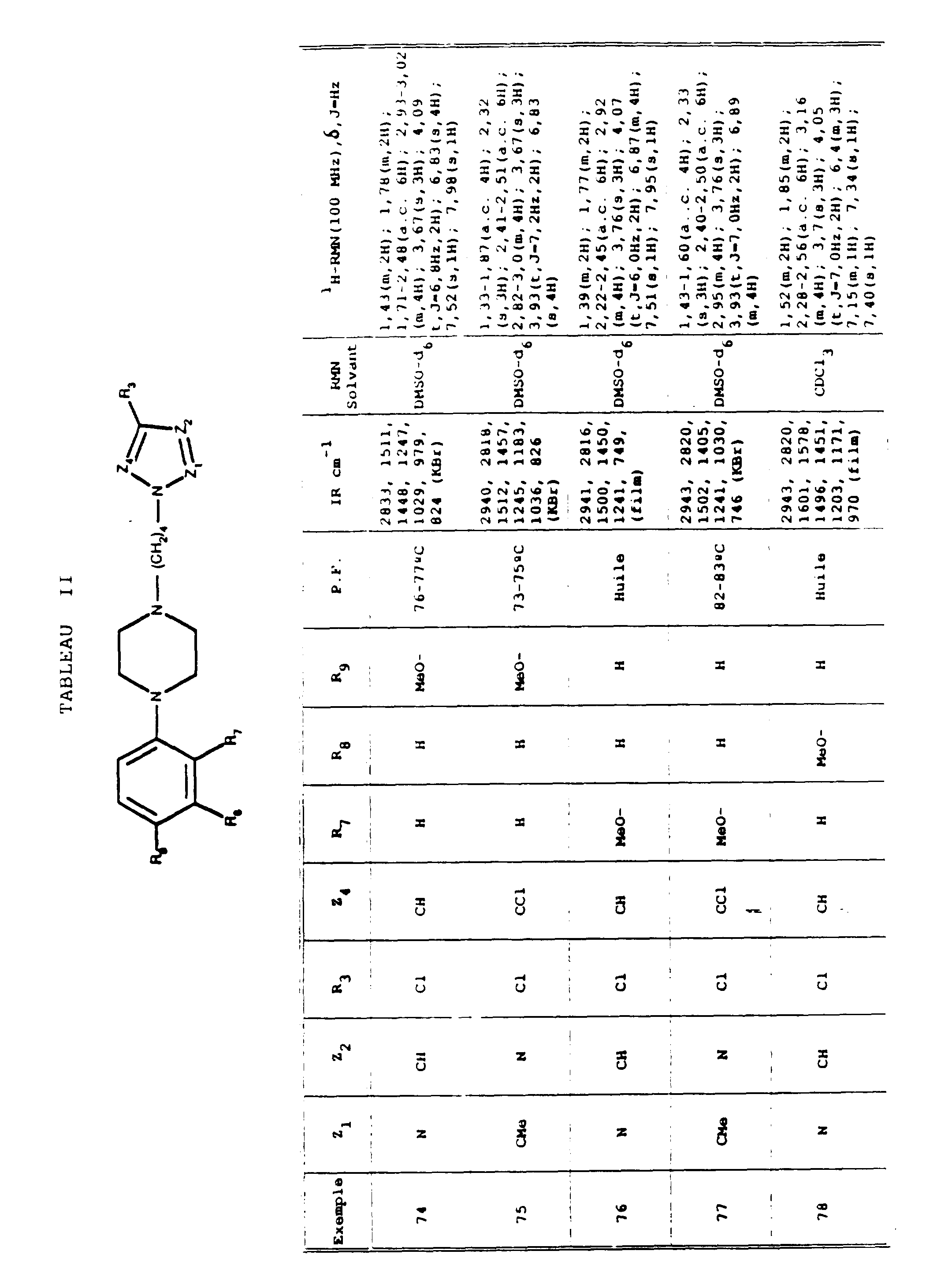

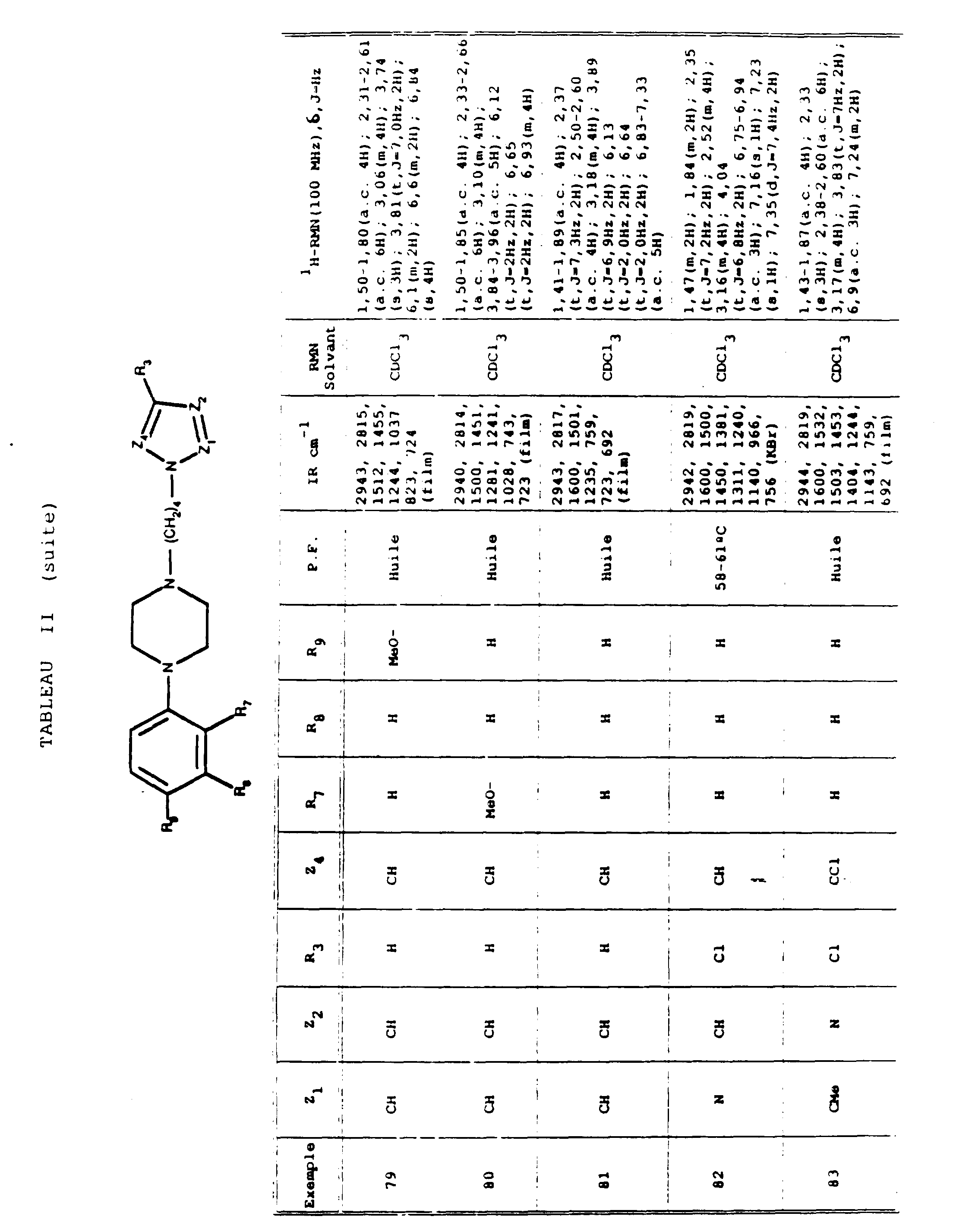

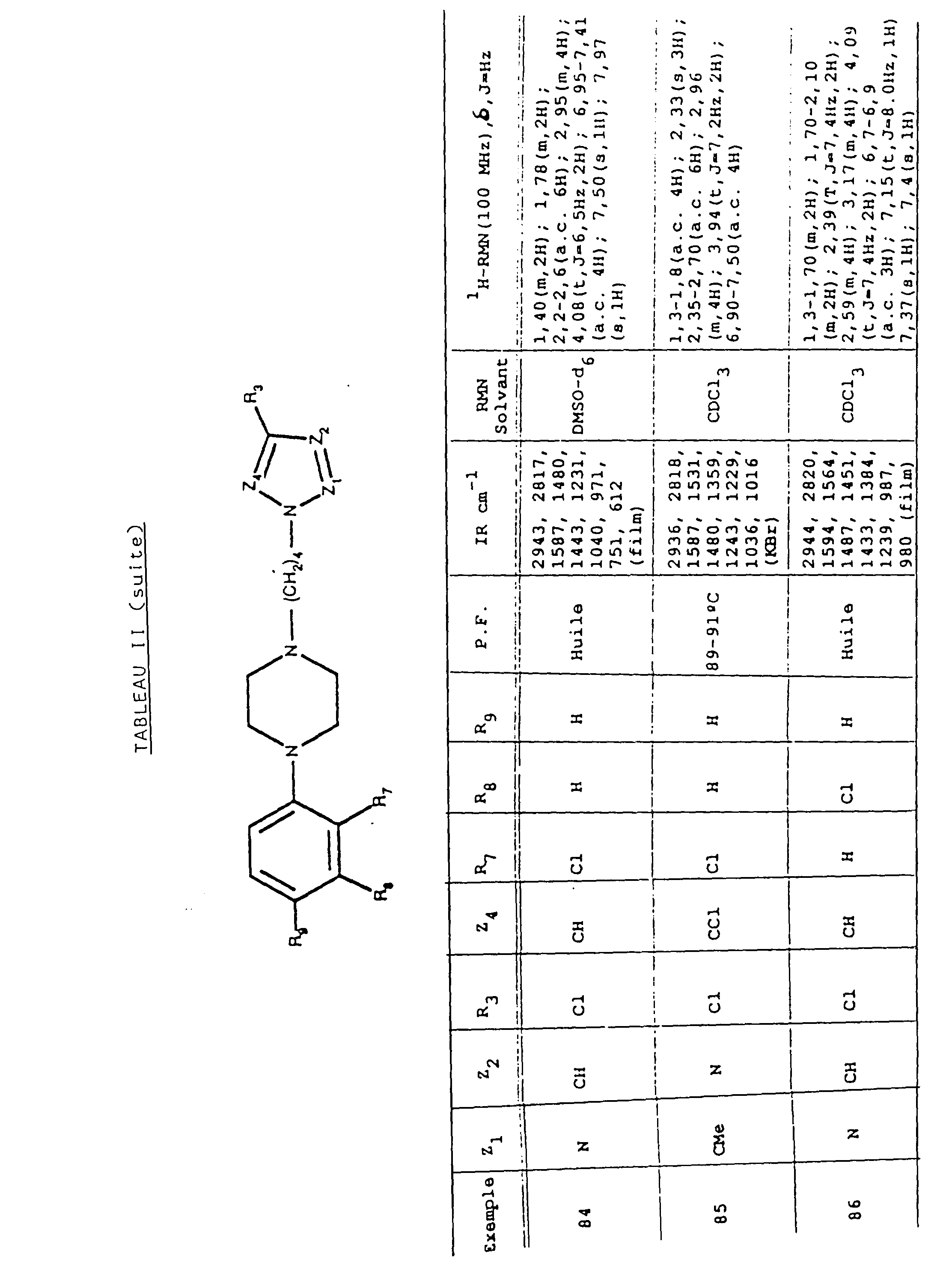

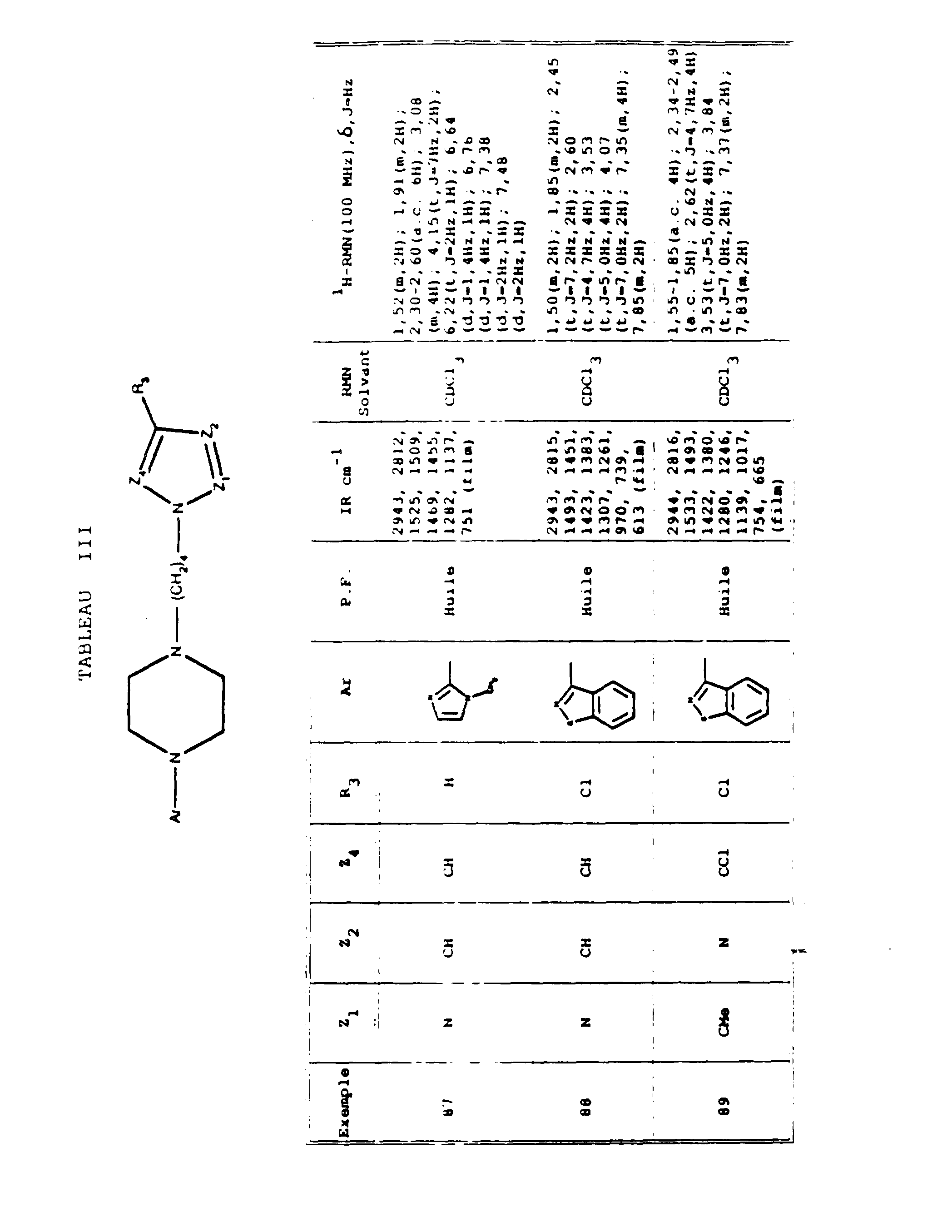

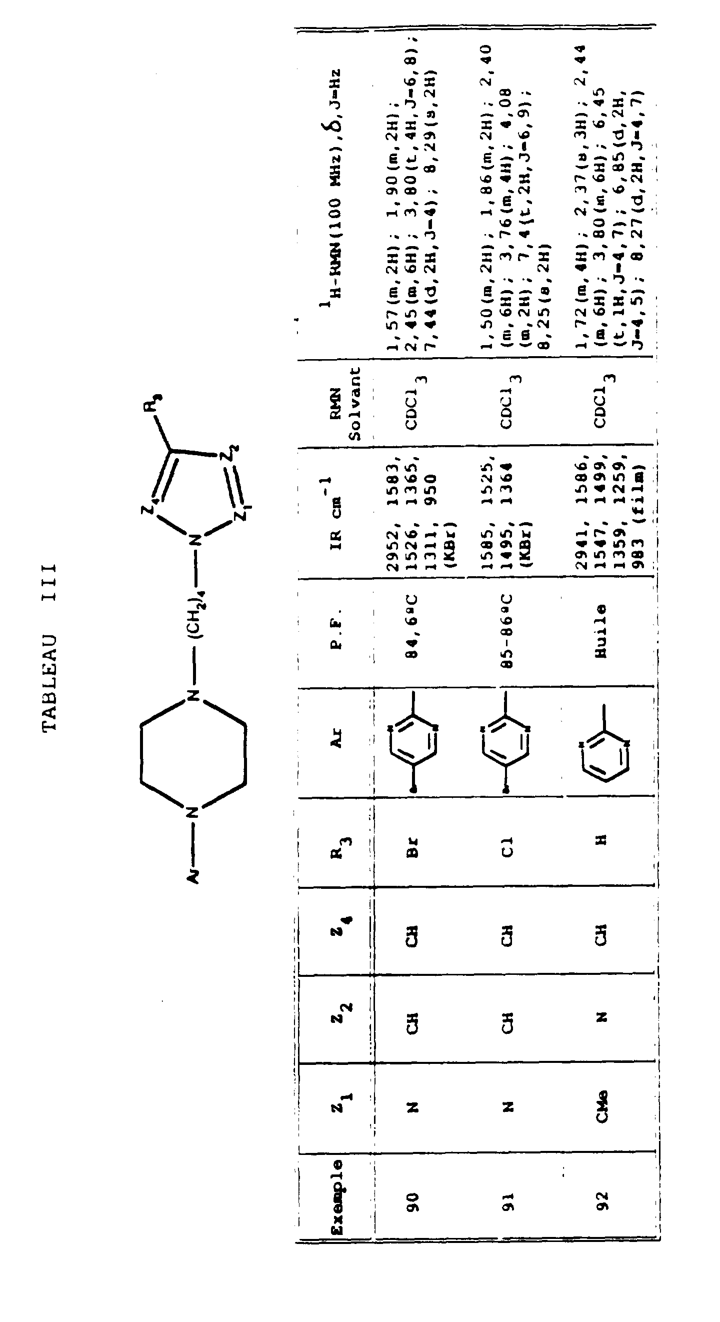

## Revendications

1. Procédé de préparation de dérivés d'aryl (ou hétéroaryl)pipérazinyl-butyl-azoles, répondant à la formule générale I 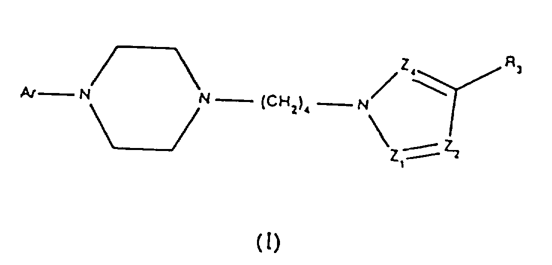 dans laquelle :
Ar représente un radical aromatique azoté ou non, choisi parmi le radical phényle, la 2-pyrimidine, le 2-N-méthylimidazole et le 3-(1,2-benzothiazole)
Z₁ représente un atome d'azote ou un atome de carbone substitué ou non qu'on peut représenter par : C-R₁
Z₂ représente un atome d'azote ou un atome de carbone substitué ou non qu'on peut représenter par: C-R₂
Z₄ représente un atome d'azote ou un atome de carbone substitué ou non qu'on peut représenter par : C-R₄
et R₁, R₂, R₃ et R₄, identiques ou différents, pouvant également former une partie d'un autre cycle, aromatique ou non, représentent un atome d'hydrogène, un halogène, un radical alkyle inférieur, un radical nitro, un radical hydroxy, un radical alcoxy, un radical cyano, un radical carboxylique, un radical carboxamido, un radical carboxylate d'alkyle, un radical aryle, un radical sulfonique, un radical sulfonamido, un radical benzamido, un radical amino.
caractérisé en ce que l'on fait réagir un composé de formule générale IX 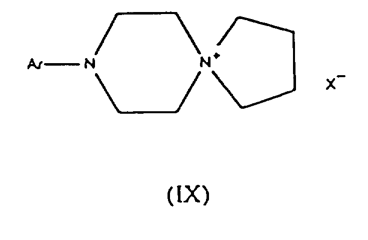 dans laquelle :
Ar a les significations mentionnées précédemment et X représente un atome d'halogène, avec un composé de formule générale III 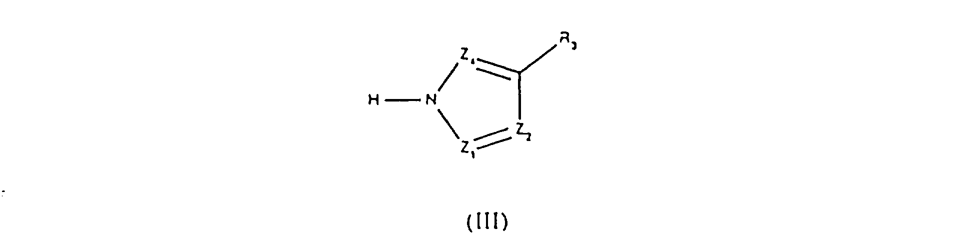 dans laquelle :
Z₁, Z₂, Z₄ et R₃ ont les significations mentionnées précédemment,
et en ce que la réaction s'effectue en présence de diméthylformamide, et de carbonate de potassium.

2. Procédé selon la revendication 1, caractérisé en ce que la réaction est mise en oeuvre à une température variant entre la température ambiante et la température d'ébullition du diméthylformamide de préférence entre 80°C et 160°C, et avec un temps réactionnel compris entre 1 heure et 24 heures.

3. Procédé de préparation des composés répondant à la formule générale I selon la revendication 1, sélectionnés parmi le groupe suivant :
1 -1-{4-[4-(2-pyrimidinyl)-1-pipérazinyl]-butyl}-pyrrole,
2 -1-{4-[4-(2-pyrimidinyl)-1-pipérazinyl]-butyl}-carbazole,
3 - 1-{4[4-(2-pyrimidinyl)-1-pipérarinyl]-butyl}-indole,
4 - 2,3-diphényl-1-{4-[4-(2-pyrimidinyl)-1-pipérazinyl]-butyl}-indole,
5 **-** 4-carboxamido-1-{4-[4-(2-pyrimidinyl)-1-pipérazinyl]-butyl}-1*H*-pyrazole,
6 - 4-carboxy-1-{4-[4-(2-pyrimidinyl)-1-pipérazinyl]-butyl}-1*H*-pyrazole.
7 - 3-méthyl-5-trifluorométhyl-1-{4-[4-(2-pyrimidinyl)-1-pipérazinyl]-butyl}-1*H*-pyrazole,
8 - 4,5-diphényl-1-{4-[4-(2-pyrimidinyl)-1-pipérazinyl]-butyl}-1*H*-imidazole,
9 -2,4,5,-triphényl-1-{4-[4-(2-pyrimidinyl)-1-pipérazinyl]-butyl}-1*H*-imidazole,
10 - 4,5-diphényl-2-méthyl-1-{4-[4-(2-pyrimidinyl)-1-pipérazinyl]-butyl}-1*H*-imidazole,
11 - 4,5,-dichloro-2-méthyl-1-{4-[4-(2-pyrimidinyl)-1-pipérazinyl]-butyl}-1*H*-imidazole,
12 - 2-éthyl-1-{4-[4-(2-pyrimidinyl)-1-pipérazinyl]-butyl}-1*H*-imidazole,
13 - 2-phényl-1-{4-[4-(2-pyrimidinyl)-1-pipérazinyl)-butyl}-1*H*-imidazole,
14 - 4-carboxylate de méthyle-1-{4-[4-(2-pydmidinyl)-1-pipérazinyl]-butyl}-1*H*-imidazole,
15 - 4-phényl-1-{4-[4-(2-pyrimidinyl)-1-pipérazinyl]-butyl}-1*H*-imidazole,
16 - 1-{4-[4-(2-pyrimidinyl)-1-pipérazinyl]-butyl}-1*H*-benzimidazole,
17 - 1-{4-[4-(2-pyrimidinyl)-1-pipérazinyl]-butyl}-3*H*-imidazo[5,4-b]pyridine,
18 - 1-{4-[4-(2-pyrimidinyl)-1-pipérazinyl]-butyl}-1*H*-imidazo[4,5-b]pyridine,
19 - 1-{4-[4-(2-pyrimidinyl)-1-pipérazinyl]-butyl}-1*H*-benzotriazole,
20 - 2-chloro-1-{4-[4-(2-pyrimidinyl)-1-pipérazinyl]-butyl}-1*H*-benzimidazole,
21 **-** 5-chloro-1-{4-[4-(2-pyrimidinyl)-1-pipérazinyl]-butyl}-1*H*-benzimidazole,
22 - 6-chloro-1-{4-[4-(2-pyrimidinyl)-1-pipérazinyl]-butyl}-1*H*-benzimidazole,
23 - 1-{4-[4-(2-pyrimidinyl)-1-pipérazinyl]-butyl}-1*H*-1,2,4-triazole,
24 - 2-{4-[4(2-pyrimidinyl)-1-pipérazinyl]-butyl}-2*H*-benzotriazole,
25 - 2-méthyl-1-{4-[4-(2-pyrimidinyl)-1-pipérazinyl]-butyl}-1*H*-benzimidazole,
26 - 5,6-diméthyl-1-{4-[4-(2-pyrimidinyl)-1-pipérazinyl]-butyl}-1*H*-benzimidazole,
27 - 1-{4-[4-(2-pyrimidinyl)-1-pipérazinyl]-butyl}-1*H*-pyrazole
28 - 3,5-diméthyl-1-{4-[4-(2-pyrimidinyl)-1-pipérarinyl]-butyl}-1*H*-pyrazole
29 - 3,5-dimethyl-4-nitro-1-{4-[4-(2-pyrimidinyl)-1-pipérazinyl]-butyl}-1*H*-pyrazole
30 - 4-methyl-1-{4-[4-(2-pyrimidinyl)-1-pipérazinyl]-butyl}-1*H*-pyrazole
31 - 1-{4-[4-(2-pyrimidinyl)-1-pipérazinyl]-butyl}-1*H*-indazole
32 - 4-bromo-3,5-diméthyl-1-{4-[4-(2-pyrimidinyl)-1-pipérazinyl]-butyl}-1*H*-pyrazole
33 - 4-nitro-1-{4-[4-(2-pyrimidinyl)-1-pipérazinyl]-butyl}-1*H*-pyrazole
34 - 4-chloro-1-{4-[4-(2-pyrimidinyl)-1-pipérazinyl]-butyl}-1*H*-pyrazole
35 - 4-carboxylate d'éthyle-1-{4-[4-(2-pyrimidinyl)-1-pipérazinyl)-butyl}-1*H*-pyrazole
36 - 3-méthyl-5-phényl-1-{4-[4-(2-pyrimidinyl)-1-pipérazinyl]-butyl}-1*H*-pyrazole
37 - 4-bromo-1-{4-[4-(2-pyrimidinyl)-1-pipérazinyl]-butyl}-1*H*-pyrazole
38 - 4-cyano-1-{4-[4-(2-pyrimidinyl)-1-pipérazinyl]-butyl}-1*H*-pyrazole
39 - 4-fluoro-1-{4-[4-(2-pyrimidinyl)-1-pipérazinyl]-butyl}-1*H*-pyrazole
40 - 4-méthoxy-1-{4-[4-(2-pyrimidinyl)-1-pipérazinyl]-butyl}-1*H*-pyrazole
41 - 4-amino-1-{4-[4-(2-pyrimidinyl)-1-pipérazinyl]-butyl}-1*H*-pyrazole
42 - 4-méthylsulfonamido-1-{4-[4-(2-pyrimidinyl)-1-pipérazinyl]-butyl}-1*H*-pyrazole
43 - 4-benzamido-1-{4-[4-(2-pyrimidinyl)-1-pipérazinyl]-butyl}-1*H*-pyrazole
44 - 4-acétamido-1-{4-[4-(2-pyrimidinyl)-1-pipérazinyl]-butyl}-1*H*-pyrazole
46 - 5-méthyl-1-{4-[4-(2-pyrimidinyl)-1-pipérazinyl]-butyl}-1*H*-pyrazole
47 - 3-méthyl-1-{4-[4-(2-pyrimidinyl)-1-pipérazinyl]-butyl}-1*H*-pyrazole
48 - 4-bromo-5-méthyl-1-{4-[4-(2-pyrimidinyl)-1-pipérazinyl]-butyl}-1*H*-pyrazole
49 - 4-bromo-3-méthyl-1-{4-[4-(2-pyrimidinyl)-1-pipérazinyl]-butyl}-1*H*-pyrazole
50 - 1-{4-[4-(2-pyrimidinyl)-1-pipérazinyl]-butyl}-1*H*-4,5,6,7-tétrahydroindazole
51 - 2-{4-[4-(2-pyrimidinyl)-1-pipérazinyl]-butyl}-1*H*-3,4,5,6-tétrahydroindazole
52 - 5-méthyl-4-phényl-1-{4-[4-(2-pyrimidinyl)-1-pipérazinyl]-butyl}-1*H*-pyrazole
53 - 3-méthyl-4-phényl-1-{4-[4-(2-pyrimidinyl)-1-pipérazinyl]-butyl}-1*H*-pyrazole
54 - 3-chloro-4-fluoro-1-{4-[4-(2-pyrimidinyl)-1-pipérazinyl]-butyl}-1*H*-pyrazole
55 - 3-chloro-4-méthoxy-1-{4-[4-(2-pyrimidinyl)-1-pipérazinyl]-butyl}-1*H*-pyrazole
58 - 4-(1-pyrrolyl)-1-{4-[4-(2-pyrimidinyl)-1-pipérazinyl]-butyl]-1*H*-pyrazole
59 - 4-phényl-1-{4-[4-(2-pyrimidinyl)-1-pipérazinyl]-butyl}-1*H*-pyrazole
60 - 3,5-diphényl-1-{4-[4-(2-pyrimidinyl)-1-pipérazinyl]-butyl}-1*H*-pyrazole
61 - 4-phénylsulfamoyl-1-{4-[4-(2-pyrimidinyl)-1-pipérazinyl]-butyl}-1*H*-pyrazole
62 - 4-(4-méthylbenzene)sulfamoyl-1-{4-[4-(2-pyrimidinyl)-1-pipérazinyl]-butyl}-1*H*-pyrazole
63 - 4-butylsulfamoyl-1-{4-[4-(2-pyrimidinyl)-1-pipérazinyl]-butyl}-1*H*-pyrazole
64 - 4-propylsulfamoyl-1-{4-[4-(2-pyrimidinyl)-1-pipérazinyl]-butyl}-1*H*-pyrazole
65 - 4-éthylsulfamoyl-1-{4-[4-(2-pyrimidinyl)-1-pipérazinyl]-butyl}-1*H*-pyrazole
66 - 3,5-diméthyl-4-(N,N-diméthylsulfonamido)-1-{4-[4-(2-pyrimidinyl)-1-pipérazinyl]-butyl}-1*H*-pyrazole
67 - 4-N-méthylsulfamoyl-1-{4-[4-(2-pyrimidinyl)-1-pipérazinyl]-butyl}-1*H*-pyrazole
68 - 4-sulfonique-1-{4-[4-(2-pyrimidinyl)-1-pipérazinyl]-butyl}-1*H*-pyrazole
69 - 1-{4-[4-(2-pyrimidinyl)-1-pipérazinyl]-butyl}-1*H*-imidazole
70 - 2-méthyl-1-{4-[4-(2-pyrimidinyl)-1-pipérazinyl]-butyl}-1*H*-imidazole
71 - 4,5-dichloro-1-{4-[4-(2-pyrimidinyl)-1-pipérazinyl]-butyl}-1*H*-imidazole
72 - 4-méthyl-1-{4-[4-(2-pyrimidinyl)-[-pipérazinyl]-butyl}-1*H*-imidazole
73 - 5-méthyl-1-{4-[4-(2-pyrimidinyl)-1-pipérazinyl]-butyl}-1*H*-imidazole
81 - 1-{4-[4-(phényl)-1-pipérazinyl]-butyl}-pyrrole,
82 - 4-chloro-1-{4-[4-(phényl)-1-pipérazinyl]-butyl}-1*H*-pyrazole,
83 - 4,5-dichloro-2-méthyl-1-{4-[4-(phényl)-1-pipérazinyl]-butyl}-1*H*-imidazole,
87 - 1-{4-[4-(2-N-méthylimidazolyl)-1-pipérazinyl]-butyl}-1*H*-pyrazole,
88 - 4-chloro-1-{4-[4-(1,2-benzisothiazol-3-yl)-1-pipérazinyl]-butyl}-1*H*-pyrazole,
89 - 4,5-dichloro-2-méthyl-1-{4-[4-(1,2-benzisothiazol-3-yl)-1-pipérazinyl]-butyl}-1*H*-imidazole,
92 - 2-méthyl-1-{4-[4-(2-pyrimidinyl)-1-pipérazinyl]-butyl}-1*H*-imidazole.

4. Procédé de préparation de dérivés d'aryl (ou hétéroaryl)-pipérazinyl-butyl-azoles, choisis parmi :
56 - 4-(4-méthoxyphényl)-1-{4-[4-(2-pyrimidinyl)-1-pipérazinyl]-butyl}-1*H*-pyrazole
57 - 4-(4-chlorophényl)-1-{4-[4-(2-pyrimidinyl)-1-pipérazinyl]-butyl}-1*H*-pyrazole
74 - 4-chloro-1-{4-[4-(4-méthoxyphényl)-1-pipérazinyl]-butyl}-1*H*-pyrazole
75 - 4,5-dichloro-2-méthyl-1-{4-[4-(4-méthoxyphényl)-1-pipérazinyl]-butyl}-1*H*-imidazole,
76 - 4-chloro-1-{4-[4-(2-méthoxyphényl)-1-pipérazinyl]-butyl}-1*H*-pyrazole,
77 - 4,5-dichloro-2-méthyl-1-{4-[4-(2-méthoxyphényl)-1-pipérazinyl]-butyl}-1*H*-imidazole,
78 - 4-chloro-1-{4-[4-(3-méthoxyphényl)-1-pipérazinyl]-butyl}-1*H*-pyrazole,
79 - 1-{4-[4-(4-méthoxyphényl)-1-pipérazinyl]-butyl}-pyrrole,
80 -1-{4-[4-(2-méthoxyphényl)-1-pipérazinyl]-butyl}-pyrrole,
84 - 4-chloro-1-{4-[4-(2-chlorophényl)-1-pipérazinyl]-butyl}-1*H*-pyrazole,
85 - 4,5-dichloro-2-méthyl-1-{4-[4-(2-chlorophényl)-1-pipérazinyl]-butyl}-1*H*-imidazole,
86 - 4-chloro-1-{4-[4-(3-chlorophényl)-1-pipérazinyl]-butyl}-1*H*-pyrazole,
90 - 4-bromo-1-{4-[4-(5-bromopyrimidin-2-yl)-1-pipérazinyl]-butyl}-1*H*-pyrazole
91 - 4-bromo-1-{4-[4-(5-chloropyrimidin-2-yl)-1-pipérazinyl]-butyl}-1*H*-pyrazole,
caractérisé en ce que l'on fait réagir un composé de formule générale IX 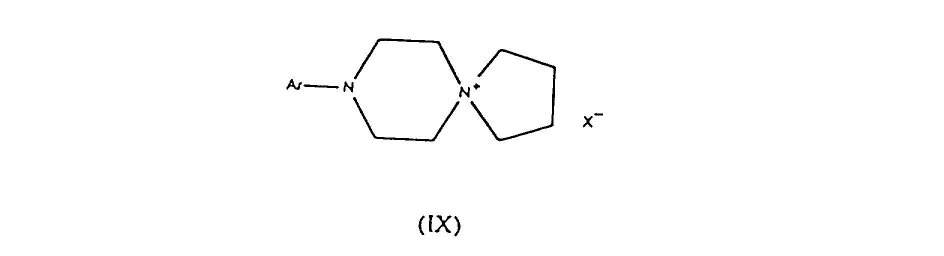 dans laquelle :
Ar a les significations correspondant aux dérivés mentionnés dans le préambule de la revendication 4 et X représente un atome d'halogène, avec un composé de formule générale III 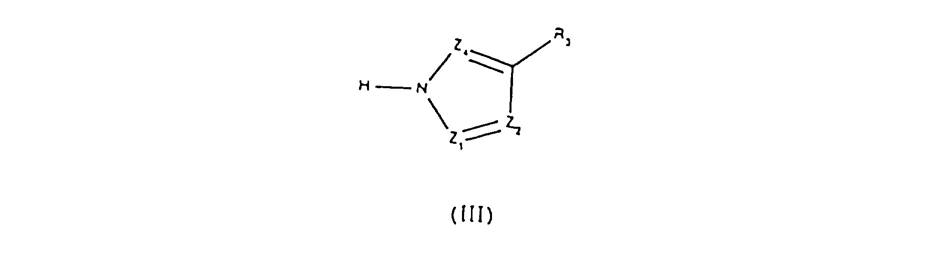 dans laquelle :
Z₁, Z₂, Z₄ et R₃ ont les significations correspondant aux dérivés mentionnés dans le préambule de la revendication 4,
et en ce que la réaction s'effectue en présence de diméthylformamide et de carbonate de potassium.

5. Procédé selon la revendication 4, caractérisé en ce que la réaction est mise en oeuve à une température variant entre la température ambiante et la température d'ébullition du diméthylformamide de préférence entre 80°C et 160°C, et avec un temps réactionnel compris entre 1 heure et 24 heures.

## Patentansprüche

1. Verfahren zur Herstellung von Aryl (oder Heteroaryl)-piperazinylbutyl-azol-Derivaten der allgemeinen Formel (I): 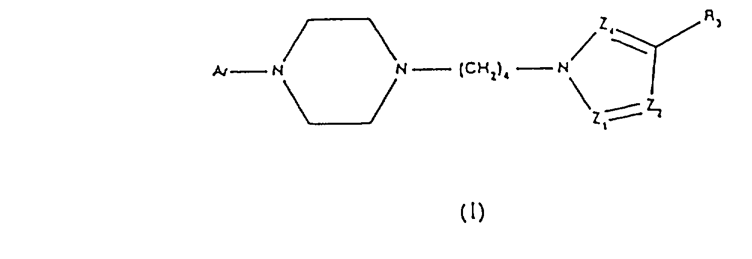 worin bedeuten:
Ar einen aromatischen Rest, der Stickstoff enthält oder nicht enthält, ausgewählt aus den Phenyl-, 2-Pyrimidin-, 2-N-Methylimidazol- und 3-(1,2-Benzothiazol)-Resten,
Z₁ ein Stickstoffatom oder ein Kohlenstoffatom, das substituiert oder unsubstituiert ist, das dargestellt werden kann durch C-R₁,
Z₂ ein Stickstoffatom oder ein Kohlenstoffatom, das substituiert oder unsubstituiert ist, das dargestellt werden kann durch C-R₂,
Z₄ ein Stickstoffatom oder ein Kohlenstoffatom, das substituiert oder unsubstituiert ist, das dargestellt werden kann durch C-R₄,
wobei R₁, R₂, R₃ und R₄, die gleich oder verschieden sind, die auch einen Teil eines anderen aromatischen oder nicht-aromatischen Ringes bilden können, ein Wasserstoffatom, ein Halogenatom, einen niederen Alkylrest, einen Nitrorest, einen Hydroxyrest, einen Alkoxyrest, einen Cyanorest, einen Carboxylrest, einen Carboxamidorest, einen Alkylcarboxylatrest, einen Arylrest, einen Sulfonsäurerest, einen Sulfonamidorest, einen Benzamidorest, einen Aminorest darstellen,
dadurch gekennzeichnet, daß man eine Verbindung der allgemeinen Formel (IX) 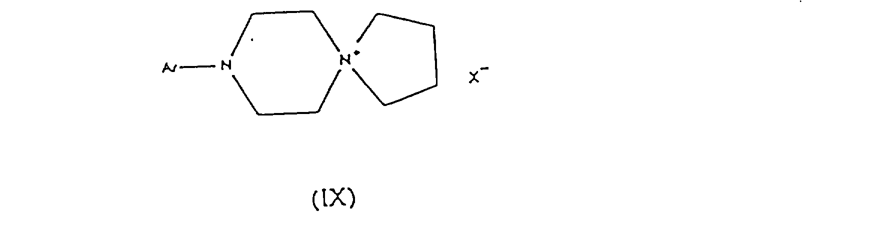
worin Ar die oben angegebenen Bedeutungen hat und X für ein Halogenatom steht,
mit einer Verbindung der allgemeinen Formel (III) reagieren läßt 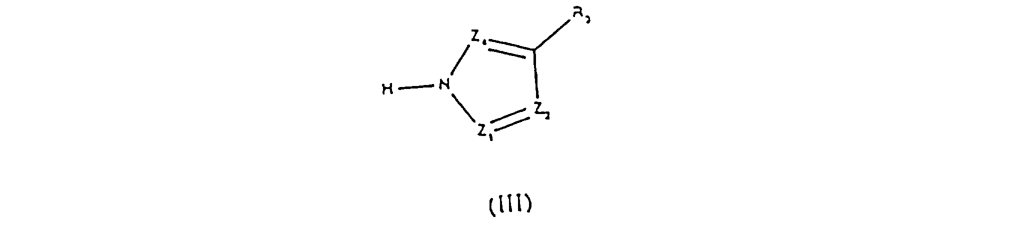 worin Z₁, Z₂, Z₄ und R₃ die oben angegebenen Bedeutungen haben, und daß man die Reaktion in Gegenwart von Dimethylformamid und Kaliumcarbonat ablaufen läßt.

2. Verfahren nach Anspruch 1, dadurch gekennzeichnet, daß die Reaktion bei einer Temperatur durchgeführt wird, die zwischen der Umgebungstemperatur und der Siedetemperatur von Dimethylformamid, vorzugsweise zwischen 80 und 160°C, variiert für eine Reaktionszeit zwischen 1 h und 24 h.

3. Verfahren zur Herstellung der Verbindungen der allgemeinen Formel (I) nach Anspruch 1, die ausgewählt werden aus der folgenden Gruppe:
1. 1-{4-[4-(2-Pyrimidinyl)-1-piperazinyl]butyl}-pyrrol,
2. 1-{4-[4-(2-Pyrimidinyl)-1 piperazinyl]butyl}carbazol,
3. 1-{4-[4-(2-Pyrimidinyl)-1-piperazinyl]butyl}-indol,
4. 2,3-Diphenyl-1-{4-[4-(2-pyrimidinyl)-1-piperazinyl]butyl}-indol,
5. 4-Carboxamido-1-{4-[4-(2-pyrimidinyl)-1-piperazinyl]butyl}-1H-pyrazol,
6. 4-Carboxy-1-{4-[4-(2-pyrimidinyl)-1-piperazinyl)butyl}-1H-pyrazol,
7. 3-Methyl-5-trifluoromethyl-1-{4-[4-(2-pyrimidinyl)-1-piperazinyl]butyl}-1H-pyrazol,
8. 4,5-Diphenyl-1-{4-[4-(2-pyrimidinyl)-1-piperazinyl]-butyl}-1H-imidazol,
9. 2,4,5-Triphenyl-1-{4-[4-(2-pyrimidinyl)-1-piperazinyl]-butyl}-1H-imidazol,
10. 4,5-Diphenyl-2-methyl-1-{4-[4-(2-pyrimidinyl)-1-piperazinyl]-butyl}-1 H-imidazol,
11. 4,5-Dichloro-2-methyl-1-{4-[4-(2-pyrimidinyl)-1-piperazinyl]-butyl}-1H-imidazol,
12. 2-Ethyl-1-{4-[4-(2-pyrimidinyl)-1-piperazinyl]-butyl}-1H-imidazol,
13. 2-Phenyl-1-{4-[4-(2-pyrimidinyl)-1-piperazinyl]-butyl}-1H-imidazol,
14. Methyl-1-{4-[4-(2-pyrimidinyl)-1-piperazinyl]-butyl}-1H-imidazol-4-carboxylat,
15. 4-Phenyl-1-{4-[4-(2-pyrimidinyl)-1-piperazinyl]-butyl}-1H-imidazol,
16. 1-{4-[4-(2-Pyrimidinyl)-1-piperazinyl]butyl}-1H-benzimidazol,
17. 1-{4-[4-(2-Pyrimidinyl)-1-piperazinyl]butyl}3H-imidazo[5,4-b]pyridin,
18. 1-{4-[4-(2-Pyrimidinyl)-1-piperazinyl]butyl}-1H-imidazo[4,5-b]pyridin,
19. 1-{4-[4-(2-Pyrimidinyl)-1-piperazinyl]butyl}-1H-benzotriazol,
20. 2-Chloro-1-{4-[4-(2-pyrimidinyl)-1-piperazinyl]-butyl}-1H-benzimidazol,
21. 5-Chloro-1-{4-[4-(2-pyrimidinyl)-1-piperazinyl]-butyl}-1H-benzimidazol,
22. 6-Chloro-1-{4-[4-(2-pyrimidinyl)-1-piperazinyl]-butyl}-1H-benzimidazol,
23. 1-{4-[4-(2-Pyrimidinyl)-1-piperazinyl]butyl}-1H-1,2,4-triazol,
24. 2-{4-[4-(2-Pyrimidinyl)-1-piperazinyl]butyl}-2H-benzotriazol,
25. 2-Methyl-1-{4-[4-(2-pyrimidinyl)-1-piperazinyl]-butyl}-1H-benzimidazol,
26. 5,6-Dimethyl-1-{4-[4-(2-pyrimidinyl)-1-piperazinyl]-butyl}-1H-benzimidazol,
27. 1-{4-[4-(2-Pyrimidinyl)-1-piperazinyl]butyl}-1H-pyrazol,
28. 3,5-Dimethyl-1-{4-[4-(2-pyrimidinyl)-1-piperazinyl]-butyl}-1H-pyrazol,
29. 3,5-Dimethyl-4-nitro-1-{4-[4-(2-pyrimidinyl)-1-piperazinyl]-butyl}-1H-pyrazol,
30. 4-Methyl-1-{4-[4-(2-pyrimidinyl)-1-piperazinyl]-butyl}-1H-pyrazol,
31. 1-{4-[4-(2-Pyrimidinyl)-1-piperazinyl]butyl}-1H-indazol,
32. 4-Bromo-3,5-dimethyl-1-{4-[4-(2-pyrimidinyl)-1-piperazinyl]-butyl}-1H-pyrazol,
33. 4-Nitro-1-{4-[4-(2-pyrimidinyl)-1-piperazinyl]-butyl}-1H-pyrazol,
34. 4-Chloro-1-{4-[4-(2-pyrimidinyl)-1-piperazinyl]-butyl}-1H-pyrazol,
35. Ethyl-1-{4-[4-(2-pyrimidinyl)-1-piperazinyl]-butyl}-1H-pyrazol-4-carboxylat,
36. 3-Methyl-5-phenyl-1-{4-[4-(2-pyrimidinyl)-1-piperazinyl]-butyl}-1H-pyrazol,
37. 4-Bromo-1-{4-[4-(2-pyrimidinyl)-1-piperazinyl]-butyl}-1H-pyrazol,
38. 4-Cyano-1-{4-[4-(2-pyrimidinyl)-1-piperazinyl]-butyl}-1H-pyrazol,
39. 4-Fluoro-1-{4-[4-(2-pyrimidinyl)-1-piperazinyl]-butyl}-1H-pyrazol,
40. 4-Methoxy-1-{4-[4-(2-pyrimidinyl)-1-piperazinyl]-butyl}-1H-pyrazol,
41. 4-Amino-1-{4-[4-(2-pyrimidinyl)-1-piperazinyl]-butyl}-1H-pyrazol,
42. 4-Methylsulfonamido-1-{4-[4-(2-pyrimidinyl)-1-piperazinyl]-butyl}-1H-pyrazol,
43. 4-Benzamido-1-{4-[4-(2-pyrimidinyl)-1-piperazinyl]-butyl}-1H-pyrazol,
44. 4-Acetamido-1-{4-[4-(2-pyrimidinyl)-1-piperazinyl]-butyl}-1H-pyrazol,
46. 5-Methyl-1-{4-[4-(2-pyrimidinyl)-1-piperazinyl]-butyl}-1H-pyrazol,
47. 3-Methyl-1-{4-[4-(2-pyrimidinyl)-1-piperazinyl]-butyl}-1H-pyrazol,
48. 4-Bromo-5-methyl-1-{4-[4-(2-pyrimidinyl)-1-piperazinyl]-butyl}-1H-pyrazol,
49. 4-Bromo-3-methyl-1-{4-[4-(2-pyrimidinyl)-1-piperazinyl]-butyl}-1H-pyrazol,
50. 1-{4-[4-(2-Pyrimidinyl)-1-piperazinyl]butyl}-1H-4,5,6,7-tetrahydroindazol,
51. 2-{4-[4-(2-Pyrimidinyl)-1-piperazinyl]butyl}-1H-3,4,5,6-tetrahydroindazol,
52. 5- Methyl-4-phenyl-1-{4-[4-(2-pyrimidinyl)-1-piperazinyl]-butyl}-1H-pyrazol,
53. 3- Methyl-4-phenyl-1-{4-[4-(2-pyrimidinyl)-1-piperazinyl]-butyl}-1H-pyrazol,
54. 3-Chloro-4-fluoro-1-{4-[4-(2-pyrimidinyl)-1-piperazinyl]-butyl}-1H-pyrazol,
55. 3-Chloro-4-methoxy-1-{4-[4-(2-pyrimidinyl)-1-piperazinyl]-butyl}-1H-pyrazol,
58. 4-(1-Pyrrolyl)-1-{4-[4-(2-pyrimidinyl)-1-piperazinyl]-butyl}-1H-pyrazol,
59. 4-Phenyl-1-{4-[4-(2-pyrimidinyl)-1-piperazinyl]-butyl}-1H-pyrazol,
60. 3,5-Diphenyl-1-{4-[4-(2-pyrimidinyl)-1-piperazinyl]-butyl}-1H-pyrazol,
61. 4-Phenylsulfamoyl-1-{4-[4-(2-pyrimidinyl)-1-piperazinyl]-butyl}-1H-pyrazol,
62. 4-(4-Methylbenzol)sulfamoyl-1-{4-[4-(2-pyrimidinyl)-1-piperazinyl]-butyl}-1H-pyrazol,
63. 4-Butylsulfamoyl-1-{4-[4-(2-pyrimidinyl)-1-piperazinyl]-butyl}-1H-pyrazol,
64. 4-Propylsulfamoyl-1-{4-[4-(2-pyrimidinyl)-1-piperazinyl]-butyl}-1H-pyrazol,
65. 4-Ethylsulfamoyl-1-{4-[4-(2-pyrimidinyl)-1-piperazinyl]-butyl}-1H-pyrazol,
66. 3,5-Dimethyl-4-(N,N-dimethylsulfonamido)-1-{4-[4-(2-pyrimidinyl)-1-piperazinyl]-butyl}-1H-pyrazol,
67. 4-N-Methylsulfamoyl-1-{4-[4-(2-pyrimidinyl)-1-piperazinyl]-butyl}-1H-pyrazol,
68. 4-Sulfonsäure-1-{4-[4-(2-pyrimidinyl)-1-piperazinyl]-butyl}-1H-pyrazol,
69. 1-{4-[4-(2-Pyrimidinyl)-1-piperazinyl]butyl}-1H-imidazol,
70. 2-Methyl-1-{4-[4-(2-pyrimidinyl)-1-piperazinyl]-butyl}-1H-imidazol,
71. 4,5-Dichloro-1-{4-[4-(2-pyrimidinyl)-1-piperazinyl]-butyl}-1H-imidazol,
72. 4-Methyl-1-{4-[4-(2-pyrimidinyl)-1-piperazinyl]-butyl}-1H-imidazol,
73. 5-Methyl-1{4-[4-(2-pyrimidinyl)-1-piperazinyl)-butyl}-1H-imidazol,
81. 1-{4-[4-(Phenyl-1-piperazinyl]-butyl}-pyrrol,
82. 4-Chloro-1-{4-[4-(phenyl)-1-piperazinyl]-butyl}-1H-pyrazol,
83. 4,5-Dichloro-2-methyl-1-{4-[4-(phenyl)-1-piperazinyl]-butyl}-1H-imidazol,
87. 1-{4-[4-(2-N-Methylimidazolyl)-1-piperazinyl]-butyl}-1H-pyrazol,
88. 4-Chloro-1-{4-[4-(1,2-benzisothiazol-3-yl)-1-piperazinyl]-butyl}-1H-pyrazol,
89. 4,5-Dichloro-2-methyl-1-{4-[4-(1,2-benzisothiazol-3-yl)-1-piperazinyl]butyl}-1H-imidazol,
92. 2-Methyl-1-{4-[4-(2-pyrimidinyl)-1-piperazinyl]-butyl}-1H-imidazol.

4. Verfahren zur Herstellung von Aryl(oder Heteroaryl) -piperazinyl-butylazol-Derivaten, die ausgewählt werden aus:
56. 4-(4-Methoxyphenyl) -1-{4-[4-(2-pyrimidinyl-1-piperazinyl]-butyl}-1H-pyrazol,
57. 4-{4-Chlorophenyl)-1-{4-[4-(2-pyrimidinyl)-1-piperazinyl]-butyl}-1H-pyrazol,
74. 4-Chloro-1-{4-[4-{4-methoxyphenyl)-1-piperazinyl]-butyl}-1H-pyrazol,
75. 4,5-Dichloro-2-methyl-1{4-[4-{4-methoxyphenyl)-1-piperazinyl]-butyl}-1H-imidazol,
76. 4-Chloro-1-{4-[4-(2-methoxyphenyl)-1-piperazinyl]-butyl}-1H-pyrazol,
77. 4,5-Dichloro-2-methyl-1-{4-[4-(2-methoxyphenyl)-1-piperazinyl]-butyl}-1H-imidazol,
78. 4-Chloro-1-{4-[4-(3-methoxyphenyl)-1-piperazinyl]-butyl}-1H-pyrazol,
79. 1-{4-[4-{4-Methoxyphenyl)-1-piperazinyl]-butyl}-pyrrol,
80. 1-{4-[4-(2-Methoxyphenyl)-1-piperazinyl]-butyl}-pyrrol,
84. 4-Chloro-1{4-[4-(2-chlorophenyl)-1-piperazinyl]-butyl}-1H-pyrazol,
85. 4,5-Dichloro-2-methyl-1-{4-[4-(2-chlorophenyl)-1-piperazinyl]-butyl}-1H-imidazol,
86. 4-Chloro-1-{4-[4-(3-chlorophenyl)-1-piperazinyl]-butyl}-1H-pyrazol,
90. 4-Bromo-1-{4-[4-(5-bromopyrimidin-2-yl)-1-piperazinyl]-butyl}-1H-pyrazol,
91. 4-Bromo-1-{4-[4-(5-chloropyrimidin-2-yl)-1-piperazinyl]-butyl}-1H-pyrazol,
dadurch gekennzeichnet, daß man eine Verbindung der allgemeinen Formel (IX) 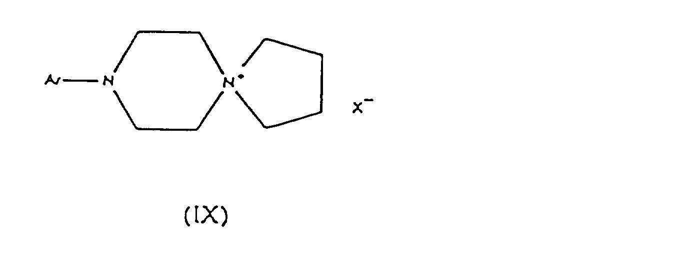 in der Ar die den im Oberbegriff dieses Anspruchs angegebenen Derivaten entsprechenden Bedeutungen hat und X für ein Halogenatom steht, mit einer Verbindung der allgemeinen Formel (III) reagieren läßt 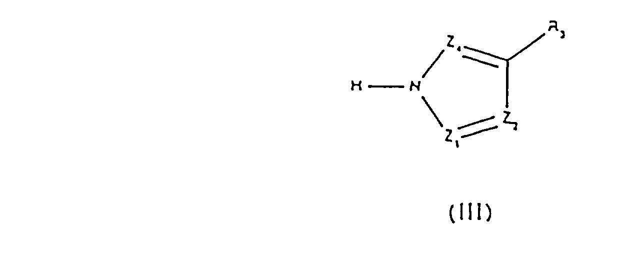 in der Z₁, Z₂, Z₄ und R₃ die den im Oberbegriff dieses Anspruchs angegebenen Derivaten entsprechenden Bedeutungen haben, und
daß man die Reaktion in Gegenwart von Dimethylformamid und Kaliumcarbonat durchführt.

5. Verfahren nach Anspruch 4, dadurch gekennzeichnet, daß die Reaktion bei einer Temperatur durchgeführt wird, die zwischen der Umgebungstemperatur und der Siedetemperatur von Dimethylformamid, vorzugsweise zwischen 80 und 160°C, variiert, bei einer Reaktionszeit zwischen 1 h und 24 h.

## Claims

1. Process for the preparation of aryl (or heteroaryl) piperazinylbutylazole derivatives corresponding to the general formula I 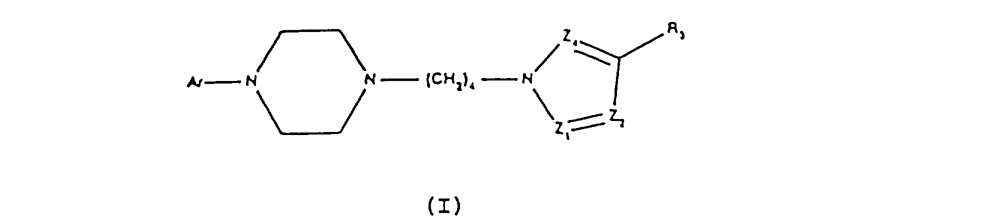 in which:
Ar denotes a nitrogenous or other aromatic radical chosen from the phenyl radical, 2-pyrimidine, 2-N-methylimidazole and 3-(1,2-benzisothiazole)
Z₁ denotes a nitrogen atom or an optionally substituted carbon atom which may be denoted by: C-R₁
Z₂ denotes a nitrogen atom or an optionally substituted carbon atom which may be denoted by: C-R₂
Z₄ denotes a nitrogen atom or an optionally substituted carbon atom which may be denoted by: C-R₄
and R₁, R₂, R₃ and R₄, which are identical or different and may also form a part of another ring, aromatic or otherwise, denote a hydrogen atom, a halogen, a lower alkyl radical, a nitro radical, a hydroxyl radical, an alkoxy radical, a cyano radical, a carboxylic radical, a carboxamido radical, an alkylcarboxylate radical, an aryl radical, a sulphonic radical, a sulphonamido radical, a benzamido radical or an amino radical.
characterized in that a compound of general formula IX 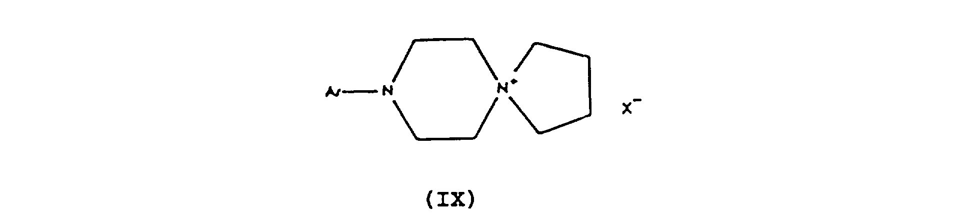 in which:
Ar has the meanings referred to above and X denotes a halogen atom, is reacted with a compound of general formula III 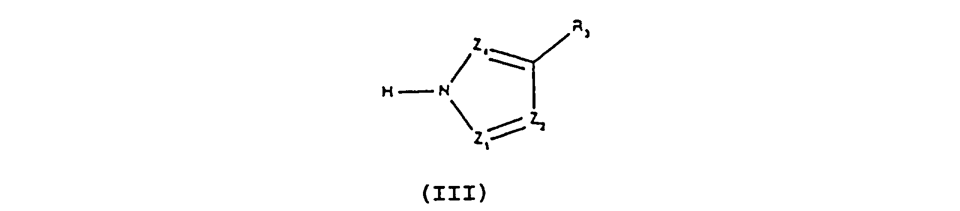 in which:
Z₁, Z₂, Z₄ and R₃ have the meanings referred to above, and in that the reaction takes place in the presence of dimethylformamide and potassium carbonate.

2. Process according to Claim 1, characterized in that the reaction is carried out at a temperature varying between room temperature and the boiling temperature of the dimethylformamide, preferably between 80°C and 160°C, and with a reaction time of between 1 hour and 24 hours.

3. Process for the preparation of the compounds corresponding to the general formula I according to Claim 1, which are selected from the following group:
1 - 1-{4-[4-(2-pyrimidinyI)-1-piperazinyl]butyl}pyrrole,
2 - 1-{4-[4-(2-pyrimidinyl)-1-piperazinyl]butyl}-carbazole,
3 - 1-{4-[4-(2-pyrimidinyl)-1-piperazinyl]butyl}indole,
4 - 2,3-diphenyl-1-{4-[4-(2-pyrimidinyl)-1-piperazinyl]-butyl}indole,
5 - 4-carboxamido-1-{4-[4-(2-pyrimidinyl)-1-piperazinyl]butyl}-1H-pyrazole,
6 - 4-carboxy-1-{4-[4-(2-pyrimidinyl)-1-piperazinyl]butyl}-1H-pyrazole,
7 - 3-methyl-5-trifluoromethyl-1-{4-[4-(2-pyrimidinyl)-1-piperazinyl]butyl}-1H-pyrazole,
8 - 4,5-diphenyl-1-{4-[4-(2-pyrimidinyl)-1-piperazinyl]butyl}-1H-imidazole,
9 - 2,4,5-triphenyl-1-{4-[4-(2-pyrimidinyl)-1-piperazinyl]butyl}-1H-imidazole,
10 - 4,5-diphenyl-2-methyl-1-{4-[4-(2-pyrimidinyl)-1-piperazinyl]butyl}-1H-imidazole,
11 - 4,5-dichloro-2-methyl-1-{4-[4-(2-pyrimidinyl)-1-piperazinyl]butyl}-1H-imidazole,
12 - 2-ethyl-1-{4-[4-(2-pyrimidinyl)-1-piperazinyl]butyl}-1H-imidazole,
13 - 2-phenyl-1-{4-[4-(2-pyrimidinyl)-1-piperazinyl]butyl}-1H-imidazole,
14 - methyl 1-{4-[4-(2-pyrimidinyl)-1-piperazinyl]butyl}-1H-imidazole-4-carboxylate,
15 - 4-phenyl-1-{4-[4-(2-pyrimidinyl)-1-piperazinyl]butyl}-1H-imidazole,
16 - 1-{4-[4-(2-pyrimidinyl)-1-piperazinyl]butyl}-1H-benzimidazole,
17 - 1-{4-[4-(2-pyrimidinyl)-1-piperazinyl]butyl}-3H-imidazo[5,4-b]pyridine,
18 - 1-{4-[4-(2-pyrimidinyl)-1-piperazinyl]butyl}-1H-imidazo[4,5-b]pyridine,
19 - 1-{4-[4-(2-pyrimidinyl)-1-piperazinyl]butyl}-1H-benzotriazole,
20 - 2-chloro-1-{4-[4-(2-pyrimidinyl)-1-piperazinyl]butyl}-1H-benzimidazole,
21 - 5-chloro-1-{4-[4-(2-pyrimidinyl)-1-piperazinyl]butyl}-1H-benzimidazole,
22 - 6-chloro-1-{4-[4-(2-pyrimidinyl)-1-piperazinyl]butyl}-1H-benzimidazole,
23 - 1-{4-[4-(2-pyrimidinyl)-1-piperazinyl]butyl}-1H-1,2,4-triazole,
24 - 2-{4-[4-(2-pyrimidinyl)-1-piperazinyl]butyl}-2H-benzotriazole,
25 - 2-methy1-1-{4-[4-(2-pyrimidinyl)-1-piperazinyl]butyl}-1H-benzimidazole,
26 - 5,6-dimethyl-1-{4-[4-(2-pyrimidinyl)-1-piperazinyl]butyl}-1H-benzimidazole,
27 -1-{4-[4-(2-pyrimidinyl)-1-piperazinyl]butyl}-1H-pyrazole
28 - 3,5-dimethyl-1-{4-[4-(2-pyrimidinyl)-1-piperazinyl]butyl}-1H-pyrazole
29 - 3,5-dimethyl-4-nitro-1-{4-[4-(2-pyrimidinyl)-1-piperazinyl]butyl}-1H-pyrazole
30 - 4-methy1-1-{4-[4-(2-pyrimidinyl)-1-piperazinyl]butyl}-1H-pyrazole
31 - 1-{4-[4-(2-pyrimidinyl)-1-piperazinyl]butyl}-1H-indazole
32 - 4-bromo-3,5-dimethyl-1-{4-[4-(2-pyrimidinyl)-1-piperazinyl]butyl}-1H-pyrazole
33 - 4-nitro-1-{4-[4-(2-pyrimidinyl)-1-piperazinyl]butyl}-1H-pyrazole
34 - 4-chloro-1-{4-[4-(2-pyrimidinyl)-1-piperazinyl]butyl}-1H-pyrazole
35 - ethyl 1-{4-[4-(2-pyrimidinyl)-1-piperazinyl]butyl}-1H-pyrazole-4-carboxylate
36 - 3-methyl-5-phenyl-1-{4-[4-(2-pyrimidinyl)-1-piperazinyl]butyl}-1H-pyrazole
37 - 4-bromo-1-{4-[4-(2-pyrimidinyl)-1-piperazinyl]butyl}-1H-pyrazole
38 -4-cyano-1-{4-[4-(2-pyrimidinyl)-1-piperazinyl]butyl}-1H-pyrazole
39 - 4-fluoro-1-{4-[4-(2-pyrimidinyl)-1-piperazinyl]butyl}-1H-pyrazole
40 - 4-methoxy-1-{4-[4-(2-pyrimidinyl)-1-piperazinyl]butyl}-1H-pyrazole
41 - 4-amino-1-{4-[4-(2-pyrimidinyl)-1-piperazinyl]butyl}-1H-pyrazole
42 - 4-methylsulphonamido-1-{4-[4-(2-pyrimidinyl)-1-piperazinyl]butyl}-1H-pyrazole
43 - 4-benzamido-1-{4-[4-(2-pyrimidinyl)-1-piperazinyl]butyl}-1H-pyrazole
44 - 4-acetamido-1-{4-[4-(2-pyrimidinyl)-1-piperazinyl]butyl}-1H-pyrazole
46 - 5-methyl-1-{4-[4-(2-pyrimidinyl)-1-piperazinyl]butyl}-1H-pyrazole
47 - 3-methyl-1-{4-[4-(2-pyrimidinyl)-1-piperazinyl]butyl}-1H-pyrazole
48 - 4-bromo-5-methyl-1-{4-[4-(2-pyrimidinyl)-1-piperazinyl]butyl}-1H-pyrazole
49 - 4-bromo-3-methyl-1-{4-[4-(2-pyrimidinyl)-1-piperazinyl]butyl}-1H-pyrazole
50 - 1-{4-[4-(2-pyrimidinyl)-1-piperazinyl]butyl}-1H-4,5,6,7-tetrahydroindazole
51 - 2-{4-[4-(2-pyrimidinyl)-1-piperazinyl]butyl}-1H-3,4,5,6-tetrahydroindazole
52 - 5-methyl-4-phenyl-1-{4-[4-(2-pyrimidinyl)-1-piperazinyl]butyl}-1H-pyrazole
53 - 3-methyl-4-phenyl-1-{4-[4-(2-pyrimidinyl)-1-piperazinyl]butyl}-1H-pyrazole
54 - 3-chloro-4-fluoro-1-{4-[4-(2-pyrimidinyl)-1-piperazinyl] butyl}-111-pyrazole
55 - 3-chloro-4-methoxy-1-{4-[4-(2-pyrimidinyl)-1-piperazinyl]butyl}-1H-pyrazole
58 - 4-(1-pyrrolyl)-1-{4-[4-(2-pyrimidinyl)-1-piperazinyl]butyl}-1H-pyrazole
59 - 4-phenyl-1-{4-[4-(2-pyrimidinyl)-1-piperazinyl]butyl}-1H-pyrazole
60 - 3,5-diphenyl-1-{4-[4-(2-pyrimidinyl)-1-piperazinyl]butyl}-1H-pyrazole
61 - 4-phenylsulphamoyl-1-{4-[4-(2-pyrimidinyl)-1-piperazinyl]butyl}-1H-pyrazole
62 - 4-{4-methylbenzene)sulphamoyl-1-{4-[4-(2-pyrimidinyl)-1-piperazinyl]butyl}-1H-pyrazole
63 - 4-butylsulphamoyl-1-{4-[4-(2-pyrimidinyl)-1-piperazinyl]butyl}-1H-pyrazole
64 - 4-propylsulphamoyl-1-{4-[4-(2-pyrimidinyl)-1-piperazinyl]butyl}-1H-pyrazole
65 - 4-ethylsulphamoyl-1-{4-[4-(2-pyrimidinyl)-1-piperazinyl]butyl}-1H-pyrazole
66 - 3,5-dimethy1-4-(N,N-dimethylsulphonamido)-1-{4-[4-(2-pyrimidinyl)-1-piperazinyl]butyl}-1H-pyrazole
67 - 4-N-methylsulphamoyl-1-{4-[4-(2-pyrimidinyl)-1-piperazinyl]butyl}-1H-pyrazole
68 - 1-{4-[4-(2-pyrimidinyl)-1-piperazinyl]butyl}-1H-pyrazole-4-sulphonic
69 - 1-{4-[4-(2-pyrimidinyl)-1-piperazinyl]butyl}-1H-imidazole
70 - 2-methyl-1-{4-[4-(2-pyrimidinyl)-1-piperazinyl]butyl}-1H-imidazole
71 - 4,5-chloro-1-{4-[4-(2-pyrimidinyl)-1-piperazinyl]butyl}-1H-imidazole
72 - 4-methyl-1-{4-[4-(2-pyrimidinyl)-1-piperazinyl]butyl}-1H-imidazole
73 - 5-methyl-1-{4-[4-(2-pyrimidinyl)-1-piperazinyl]butyl}-1H-imidazole
81 - 1-{4-[4-(phenyl)-1-piperazinyl]butyl}pyrrole,
82 - 4-chloro-1-{4-[4-(phenyl)-1-piperazinyl]butyl}-1H-pyrazole,
83 - 4,5-dichloro-2-methyl-1-{4-[4-(phenyl)-1-piperazinyl]butyl}-1H-imidazole,
87 - 1-{4-[4-(2-N-methylimidazolyl)-1-piperazinyl]butyl}-1H-pyrazole,
88 -4-chloro-1-{4 [4-(1,2-benzisothiazol-3-yl)-1-piperazinyl]butyl}-1H-pyrazole,
89 - 4,5-dichloro-2-methyl-1-{4-[4-(1,2-benzisothiazol-3-yl)-1-piperazinyl]butyl}-1H-imidazole,
92 - 2-methyl-1-{4-[4-(2-pyrimidinyl)-1-piperazinyl]butyl}-1H-imidazole.

4. Process for the preparation of aryl (or heteroaryl) piperazinylbutylazole derivatives, which are chosen from:
56 - 4-(4-methoxyphenyl)-1-{4-[4-(2-pyrimidinyl)-1-piperazinyl]butyl}-1H-pyrazole
57 - 4-(4-chlorophenyl)-1-{4-[4-(2-pyrimidinyl)-1-piperazinyl]butyl}-1H-pyrazole
74 - 4-chloro-1-{4-[4-{4-methoxyphenyl)-1-piperazinyl]butyl}-1H-pyrazole
75 - 4,5-dichloro-2-methyl-1-{4-[4-{4-methoxyphenyl)-1-piperazinyl]butyl}-1H-imidazole,
76 - 4-chloro-1-{4-[4-(2-methoxyphenyl)-1-piperazinyl]butyl}-1H-pyrazole,
77 - 4,5-dichloro-2-methyl-1-{4-[4-(2-methoxyphenyl)-1-piperazinyl]butyl}-1H-imidazole,
78 - 4-chloro-1-{4-[4-(3-methoxyphenyl)-1-piperazinyl]butyl}-1H-pyrazole,
79 - 1-{4-[4-(4-methoxyphenyl)-1-piperazinyl]butyl}pyrrole,
80 - 1-{4-[4-(2-methoxyphenyl)-1-piperazinyl]butyl}pyrrole,
84 - 4-chloro-1-{4-[4-(2-chlorophenyl)-1-piperazinyl]butyl}-1H-pyrazole,
85 - 4,5-dichloro-2-methyl-1-{4-[4-(2-chlorophenyl)-1-piperazinyl]butyl}-1H-imidazole,
86 - 4-chloro-1-{4-[4-(3-chlorophenyl)-1-piperazinyl]butyl}-1H-pyrazole,
90 - 4-bromo-1-{4-[4-(5-bromopyrimidin-2-yl)-1-piperazinyl]butyl}-1H-pyrazole
91 - 4-bromo-1-{4-[4-(5-chloropyrimidin-2-yl)-1-piperazinyl]butyl}-1H-pyrazole,
characterized in that a compound of general formula IX 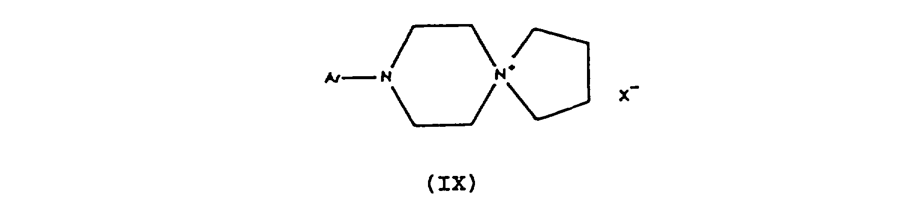 in which:
Ar has the meanings corresponding to the derivatives referred to in the preamble to Claim 4 and X denotes a halogen atom,
is reacted with a compound of general formula III 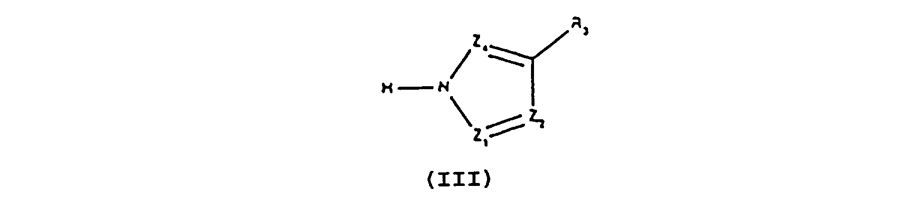 in which:
Z₁, Z₂, Z₄ and R₃ have the meanings corresponding to the derivatives referred to in the preamble to Claim 4, and in that the reaction takes place in the presence of dimethylformamide and potassium carbonate.

5. Process according to Claim 4, characterized in that the reaction is carried out at a temperature varying between room temperature and the boiling temperature of the dimethylformamide, preferably between 80°C and 160°C, and with a reaction time of between 1 hour and 24 hours.
